# FASCICULE DE BREVET EUROPEEN

(11) **EP 0 615 978 B1**
(45) Date de publication et mention de la délivrance du brevet: **12.05.1999**
(21) Numéro de dépôt: 94400393.8
(22) Date de dépôt: 24.02.1994
(51) Int. Cl.: C07K 5/06, A61K 38/55, C07F 5/02

(54) **Derivés peptidiques de l'acide boronique ayant une activité inhibitant de protéase, leur procédé de préparation et les compositions pharmaceutiques qui les contiennent**
Peptidderivate aus Boronsäure mit Protease-inhibierender Aktivität, ihr Herstellungsverfahren und sie enthaltende pharmazeutische Zusammensetzungen
Peptide derivatives of the boronic acid having protease inhibiting activity, process for their production and pharmaceutical compositions comprising them

(30) Priorité: 24.02.1993 FR 9302082
(43) Date de publication de la demande: 21.09.1994
(73) Titulaire: ADIR ET COMPAGNIE, 92415 Courbevoie Cédex (FR)
(72) Inventeur: de Nanteuil, Guillaume, F-92150 Suresnes (FR); Lila, Christine, F-78220 Viroflay (FR); Laubie, Michel, F-92420 Vaucresson (FR); Verbeuren, Tony, F-78540 Vernouillet (FR); Simonet, Serge, F-78700 Conflans Sainte Honorine (FR); Rupin, Alain, F-37510 Savonnieres (FR); Portevin, Bernard, F-78990 Elancourt (FR)

(56) Documents cités:
- EP-A- 0 315 574
- EP-A- 0 471 651
- WO-A-92/07869
- FR-A- 2 694 295
- TETRAHEDRON LETTERS vol. 33, no. 29 , 14 Juillet 1992 , OXFORD GB pages 4209 - 4212 S. ELGENDY ET AL 'New Peptide Boronic Acid Inhibitors of Thrombin'

## Description

La présente invention concerne des dérivés peptidiques d'acide boronique, leur procédé de préparation, les compositions pharmaceutiques qui les contiennent ainsi que leur utilisation en tant qu'inhibiteurs de trypsine-like serine proteases.

L'une de ces serine proteases, la thrombine, est l'enzyme clé de la coagulation et joue un rôle central dans la pathologie des thromboses veineuses et artérielles comme l'ont montré F. Toti et coll. (Sang, Thrombose, Vaisseaux, 4, 483494, 1992) et T.M. REILLY et coll. (Blood Coagulation and Fibrinolysis, 3, 513-517, 1992).

Les approches anti-thrombotiques sont plus efficaces et sans risque par rapport aux traitements actuels. Des inhibiteurs directs de la thrombine, actuellement en développement clinique, présentent toute une série d'avantages sur l'héparine. Mais ces substances, l'hirudine et l'hirulog-1 ont le désavantage de ne pas être actives par voie orale.

D'autre part, il est connu que des peptides contenant la séquence (D)Phe-Pro-Arg présente dans le fibrinogène, sont des inhibiteurs du site catalytique de la thrombine (C. KETTNER et coll., J. Biol. Chem., 265 (30), 18289-18297, 1990).

Des dérivés peptidiques de l'acide boronique, présentant une activité anti-thrombotique, ont déjà été décrits dans la littérature. C'est le cas plus particulièrement des composés décrits dans les brevets EP 293881 et EP 471651. M.A. HUSSAIN et coll. ont d'ailleurs démontré que l'acide Ac-(D)Phe-Pro-Arg boronique (DUP 714) est un inhibiteur de la thrombine (Peptides, 12, 1153-1154, 1991).

Il était donc particulièrement intéressant de synthétiser de nouveaux inhibiteurs de serine proteases afin d'augmenter la puissance, la selectivité ainsi que l'activité par voie orale des composés déjà décrits dans la littérature.

Plus spécifiquement, le présente invention concerne les composés de formule (I) : 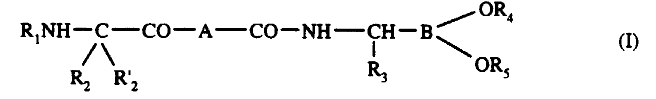 dans laquelle:
- R₁: représente un atome d'hydrogène ou un groupement acyle (C₁-C₆) linéaire ou ramifié, alkyle (C₁-C₆) linéaire ou ramifié, benzyle, alkoxycarbonyle (C₁-C₆) linéaire ou ramifié, benzyloxycarbonyle, phénoxycarbonyle, 5-[(diméthyl)amino]naphtylsulfonyl, ou alkoxycarbonylméthyle ou carboxyméthyle,
- R₂: représente un atome d'hydrogène ou un groupement :
- phényle,
- benzyle (substitué ou non sur le noyau phényle par un ou plusieurs atomes d'halogène ou groupements alkyle (C₁-C₆) linéaire ou ramifié, alkoxy (C₁-C₆) linéaire ou ramifié, hydroxy ou bicyclooct-1-yle (substitué ou non par un groupement hydroxy ou alkoxy (C₁-C₆) linéaire ou ramifié)),
- thiénylméthyle,
- (pyridinyl)méthyle,
- diphénylméthyle,
- fluorényle,
- naphtylméthyle,
- benzocyclobutyle,
- (dicyclopropylméthyl)méthyle,
- indanyle,
- ou, cycloalkyl (C₃-C₇) méthyle,
- R'₂: représente un atome d'hydrogène ou un groupement benzyle
ou bien
- R₂ et R'₂: représentent ensemble C₆H₅-CH=,
- R₃: représente un groupement :
- alkyle (C₂-C₄) linéaire ou ramifié substitué par un groupement alkoxy (C₁-C₆) linéaire ou ramifié, phénoxy, guanidino, amidino, amino, 2-tétrahydrofuryle ou cycloalkyle (C₃-C₇),
- guanidinophényle, amidinophényle, aminophényle,
- guanidinobenzyle, amidinobenzyle, aminobenzyle,
- ou, cycloalkyle (C₃-C₇),
- R₄ et R₅: représentent chacun un atome d'hydrogène ou un groupement alkyle (C₁-C₆) linéaire ou ramifié,
ou 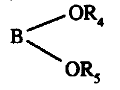 forme un ester boronique de pinanediol,
- A: représente l'un quelconque des groupements suivants :
- 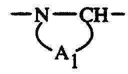 dans lequel :
   A₁ représente avec les atomes d'azote et de carbone avec lesquels il est lié une structure cyclique choisie parmi les structures suivantes :
   - perhydroindole,
   - 2-azabicyclo[2.2.2]octane,
   - 2-azabicyclo[3.3.0]octane,
   - 2-azabicyclo[2.2.1]heptane,
   - perhydroisoindole,
   - indoline,
   - isoindoline,
   - perhydroquinoléine,
   - perhydroisoquinoléine,
   - 1,2,3,4-tétrahydroquinoléine,
   - 1,2,3,4-tétrahydroisoquinoléine,
   - thiazolidine éventuellement substituée par un ou plusieurs groupements alkyl (C₁-C₆) linéaire ou ramifié,
- 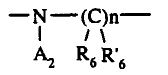 dans lequel :
   n représente 1 ou 2,
   R₆ représente un atome d'hydrogène ou un groupement cycloalkyle (C₃-C₇), alkyle (C₁-C₆) linéaire ou ramifié ou carboxyméthyle,
   R'₆ représente un atome d'hydrogène, ou bien
   R₆ et R'₆ forment avec l'atome de carbone qui les portent un groupement cycloalkyle (C₃-C₇),
   A₂ représente un groupement alkyle (C₁-C₆) linéaire ou ramifié, phényle, indanyle, cycloalkyle (C₃-C₇) (substitué ou non par un ou plusieurs groupements méthyle) cycloalkényle (C₃-C₇), cyclopentadiènyle, 2,2,2,-trifluoro-1-cyclopentyléthyl, bicyclo[2.1.1]hexyl, bicyclo[2.2.1]heptyl ou un groupement : 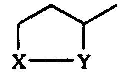 dans lequel
   X et Y différents représentent un atome d'oxygène, de soufre, un groupement NH ou CH₂, ou bien
   A₂ représente un atome d'hydrogène (à la condition que dans ce cas R₆ représente un groupement cycloalkyle (C₃-C₇) et R'₆ représente un atome d'hydrogène, ou bien R₆ et R'₆ forment avec l'atome de carbone qui les portent un groupement cycloalkyle (C₃-C₇)),
leurs énantiomères, diastéréoisomères et épimères ainsi que leurs sels d'addition à un acide ou à une base pharmaceutiquement acceptable.

L'invention concerne également le procédé de préparation des dérivés de formule (I) caractérisé en ce que l'on fait réagir un amino-acide protégé de formule (II), dont on a éventuellement séparé les isomères par une technique classique de séparation : 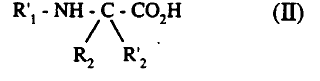 dans laquelle:
- R'₁: représente un groupement acyle (C₁-C₆) linéaire ou ramifié, benzyle, aikoxycarbonyle (C₁-C₆) linéaire ou ramifié, benzyloxycarbonyle ou phénoxycarbonyle,
- R₂ et R'₂: ont la même signification que dans la formule (I),
que l'on fait réagir selon la technique de couplage peptidique décrite par W. KONIG et R. GEIGER (Ber., 103, 788, 1970), avec un deuxième amino-acide protégé de formule (III) dont on a éventuellement séparé les isomères selon une technique classique de séparation,

HA-CO₂CH₂C₆H₅ (III)

dans laquelle A a la même signification que dans la formule (I), pour conduire au composé de formule (IV) : 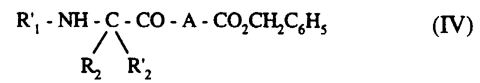 dans laquelle R'₁, R₂, R'₂ et A ont la même signification que précédemment,
dont on déprotège la fonction acide par hydrogénation catalytique pour conduire au composé de formule (V) : 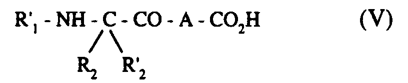 dans laquelle R'₁, R₂, R'₂ et A ont la même signification que précédemment,
que l'on met en réaction avec du N-hydroxysuccinimide en présence de 1,3-dicyclohexylcarbodiimide en milieu anhydre, pour conduire au composé de formule (VI) : 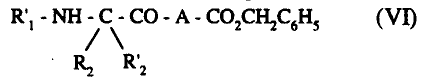 dans laquelle R'₁, R₂, R'₂ et A ont la même signification que précédemment et Suc représente un radical succinimido,
que l'on met en réaction en milieu basique avec un composé de formule (VII) : 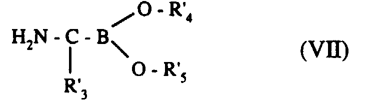 dans laquelle :
- R'₃: représente un groupement :
- alkyle (C₂-C₄) linéaire ou ramifié substitué par un atome d'halogène ou un groupement alkoxy (C₁-C₆) linéaire ou ramifié, phénoxy, 2-tétrahydrofuryle ou cycloalkyle,
- cycloalkyle (C₃-C₇),
- phényle ou benzyle, chacun étant substitué par un atome d'halogène ou un groupement amino convenablement protégé,
- R'₄ et R'₅: représente chacun un groupement alkyle (C₁-C₆) linéaire ou ramifié,
ou 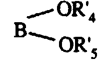 forme un ester boronique de pinanediol,
pour conduire :
- soit au composé de formule (I/a), cas particulier des composés de formule (I) : 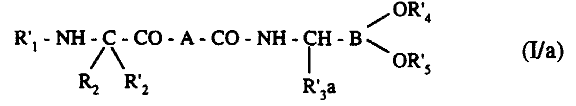 dans laquelle R'₁, R₂, R'₂, A, R'₄ et R'₅ ont la même signification que précédemment et R'_{3ₐ} représente un groupement alkyle (C₂-C₄) linéaire ou ramifié (substitué par un groupement alkoxy (C₁-C₆) linéaire ou ramifié, phénoxy, 2-tétrahydrofuryle ou cycloalkyle (C₃-C₇)) ou un groupement cycloalkyle (C₃-C₇),
- soit au composé de formule (VIII) : 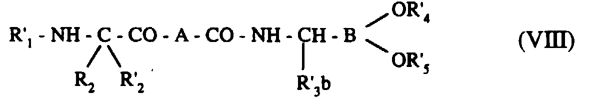 dans laquelle R'₁, R₂, R'₂, A, R'₄ et R'₅ ont la même signification que précédemment et R'_{3b} représente un groupement :
   - alkyle (C₂-C₄) substitué par un atome d'halogène,
   - phényle ou benzyle, chacun étant substitué par un atome d'halogène ou un groupement amino convenablement protégé,
composé de formule (VIII) :
° qui lorsque R'_{3b} représente un groupement :
   - alkyle substitué par un atome d'halogène,
   - phényle ou benzyle, chacun étant substitué par un atome d'halogène,
   peut être transformé en dérivé cyano correspondant par action du cyanure de cuivre puis en dérivé amidino correspondant de formule (I/b), cas particulier des composés de formule (I), par l'action de l'ammoniaque : 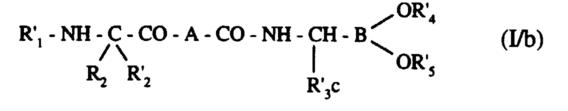 dans laquelle R'₁, R₂, R'₂, A, R'₄ et R'₅ ont la même signification que précédemment et R'_{3_{c}} représente un groupement alkyle, phényle ou benzyle, chacun étant substitué par un groupement amidino,
° qui lorsque R'_{3b} représente :
   - un groupement alkyle substitué par un atome d'halogène peut être transformé en dérivé azido correspondant par action de l'azoture de sodium, puis par hydrogénation catalytique en dérivé amino correspondant de formule (I/c), cas particulier des composés de formule (I),
   - ou, un groupement phényle ou benzyle, chacun étant substitué par un groupement amino protégé,
      peut subir une déprotection pour conduire au composé de formule (I/c), cas particulier des composés de formule (I) : 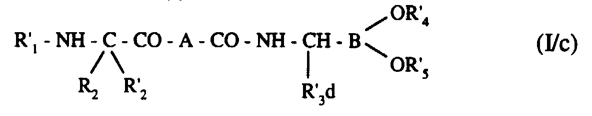
   dans laquelle R'₁, R₂, R'₂, A, R'₄ et R'₅ ont la même signification que précédemment et R'_{3d} représente un groupement alkyle, phényle ou benzyle, chacun étant substitué par un groupement amino,
composé de formule (I/c),
dont on peut transformer le radical amino du groupement R'_{3d} en radical guanidino par réaction avec du cyanamide, pour conduire au composé de formule (I/d), cas particulier des composés de formule (I) : 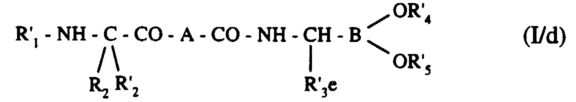 dans laquelle R'₁, R₂, R'₂, A, R'₄ et R'₅ ont la même signification que précédemment et R'₃ₑ représente un groupement alkyle, phényle ou benzyle, chacun étant substitué par un groupement guanidino,
composé de formule (I/a), (I/b), (I/c) ou (I/d) dont on déprotège, si on le souhaite, la fonction amine terminale et que l'on transforme, en milieu inerte, à l'aide de trichlorure de bore, en acide bororique de formule (I/e), cas particuler des composés de formule (I) : 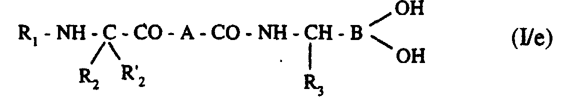 dans laquelle R₁, R₂, R'₂, A et R₃ ont la même signification que dans la formule (I),
composé de formule (I/a), (I/b), (I/c), (I/d) ou (I/e) :
- que l'on purifie éventuellement selon une technique classique de purification,
- dont on sépare, si on le souhaite, les énantiomères selon une technique classique de séparation
et que l'on transforme, le cas échéant, en ses sels d'addition à un acide ou une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, oxalique, etc...

Parmi les bases pharmaceutiquement acceptables, on peut citer à titre non limitatif, l'hydroxyde de sodium, l'hydroxyde de potassium, le bicarbonate de sodium, etc...

Les composés de formule (VII) sont obtenus :
- soit à partir du composé de formule (IX) obtenu selon le procédé décrit par M.W. RATHKE et coll. (J. Organomet.. Chem., 112, 145-149, 1976) : 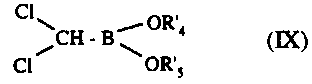 dans laquelle R'₄ et R'₅ sont tels que définis plus haut,
   que l'on fait réagir sur un organomagnésien de formule (X) :

   RMgCl (X)

   dans laquelle R représente :
   - un groupement alkyl (C₂-C₄) linéaire ou ramifié (substitué par un groupement alkoxy (C₁-C₆) linéaire ou ramifié, phénoxy, 2-tétrahydrofuryl ou cycloalkyl (C₅-C₇)),
   - un groupement cycloalkyl (C₃-C₇),
   - ou, un groupement phényl ou benzyl, chacun étant substitué par un groupement amino convenablement protégé ou par un atome d'halogène,

   pour conduire au composé de formule (XI) : 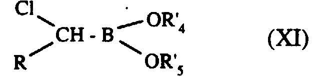 dans laquelle R, R'₄ et R'₅ sont tels que définis précédemment, que l'on met en réaction avec le 1,1,1,3,3,3-hexaméthyldisilazane (HMDS) en présence de n-butyllithium pour conduire, après traitement en milieu acide, au composé de formule (VII),
- soit à partir d'un ester boronique α-chloré de formule (XII), préparé selon le procédé décrit par D.S. MATTESON et coll. (Organometallics, 3, 1284-1288, 1984) et W. RATTHKE et coll. (J. Biol. Chem., 265 (30), 18289-18297, 1990) : 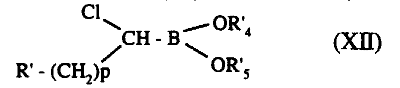 dans laquelle R'₄ et R'₅ sont tels que définis plus haut, p est un entier égal à 3 ou 4 et R' représente un atome d'halogène ou groupement alkoxy, que l'on met en réaction avec le 1,1,1,3,3,3-hexaméthyldisilazane (HMDS) en présence de n-butyllithium pour conduire, après traitement en milieu acide, au composé de formule (VII).

Les composés de la présente invention, outre le fait qu'ils soient nouveaux, présentent des propriétés pharmacologiques particulièrement intéressantes.

Ce sont de puissants inhibiteurs de trypsine-like serine proteases qui présentent une importante selectivité vis-à-vis de la thrombine par rapport à d'autres serine proteases de la coagulation. lls possèdent d'autre part une meilleure activité par voie orale que le composé de référence DUP 714.

Ces propriétés les rendent donc utiles dans le traitement des angines stables ou non, des maladies d'origine thrombotique et/ou donnant lieu à des complications thrombotiques ainsi que dans le traitement ou la prévention de l'infarctus du myocarde et des thromboses veineuses ou artérielles.

Ils peuvent également être utilisés en association thérapeutique avec un thrombolytique.

L'invention s'étend aussi aux compositions pharmaceutiques renfermant comme principe actif au moins un composé de formule (I) avec un ou plusieurs excipients inertes, non toxiques et appropriés. Les compositions pharmaceutiques ainsi obtenues pourront être présentées sous diverses formes, les plus avantageuses étant les comprimés, les dragées, les gélules, suppositoires, suspensions buvables, etc...

La posologie utile est adaptable selon la nature et la sévérité de l'affection, la voie d'administration ainsi que selon l'âge et le poids du patient. Cette posologie varie de 1 à 500 mg par jour en une ou plusieurs prises.

Les exemples suivants illustrent l'invention mais ne la limitent en aucune façon.

Les produits de départ utilisés sont des produits de départ connus ou préparés selon des modes opératoires connus.

Les préparations A, B et C conduisent à des intermédiaires utiles dans la préparation des composés de l'invention.

### Préparation A : (R)-1-Amino-4-bromobutylboronate de (+)α-pinanediol, chlorhydrate

Ce composé a été obtenu selon le procédé décrit par C. KETTNER et coll. (J. Biol. Chem., 265 (30), 18289-18297, 1990) par réaction du bromure d'allyle sur le catécholborane, suivie d'une transestérification avec du (+)α-pinanediol puis d'une réaction d'homologation en présence de dichlorométhyllithium et enfin de la réaction avec de l'hexaméthyl-disilazane.
Point de fusion : 160°C
Pouvoir rotatoire : [α]_{D}²⁵ = + 16,5° (c = 1 %, éthanol)

| Microanalyse élémentaire : | | | | |
|---|---|---|---|---|
| | C % | H % | N % | Cl % |
| calculé | 45,88 | 7,15 | 3,82 | 9,67 |
| trouvé | 45,82 | 7,09 | 4,13 | 9,85 |

### Préparation B : (R)-(1-Amino-1-cyclopentyl)méthylboronate de (+)α-pinanediol, chlorhydrate

### Stade A : Dichlorométhylboronate de (+)α-pinanediol

Ce composé a été synthétisé selon le procédé décrit par W. RATHKE et coll. (J. Organomet. Chem., 122, 145-149, 1976) à partir de dichlorométhane et de (+)α-pinanediol et est purifié par distillation sous vide.
Point d'ébullition : 125°C (p = 0,4 mm/Hg)

### Stade B : Cyclopentyl chlorométhylboronate de (+)α-pinanediol

A une solution contenant 4,75 mmoles du composé obtenu au stade précédent dans 7 ml de tétrahydrofurane anhydre refroidie à -78°C sont ajoutées, goutte à goutte, 4,75 mmoles de chlorure de cyclopentylmagnésium. L'ensemble est maintenu une heure à 0°C, puis après retour à température ammbiante, le THF est évaporé. Le résidu est repris par un mélange éther-eau, la phase éthérée décantée, séchée et évaporée et conduit au produit attendu qui est purifié par chromatographie sur colonne de silice en utilisant comme solvant un mélange pentane/dichlorométhane (90/10).

### Stade C : (R)-(1-Amino-1-cyclopentyl)méthylboronate de (+)α-pinanediol, chlorhydrate

Ce composé a été obtenu selon le procédé décrit par C. KETTNER et coll. (J. Biol. Chem., 265 (30), 18289-18297, 1990) en utilisant le composé décrit au stade précédent.
Pouvoir rotatoire : [α]_{D}²¹ = + 13,4° (c = 1 %, méthanol)

### Préparation C : (R)-1-Amino-4-méthoxybutylboronate (+)α-pinanediol, chlorhydrate

### Stade A : Allylméthyléther

93 mmoles de méthylate de sodium et 85 mmoles de bromure d'allyle sont agitées dans une bouteille pression à température ambiante pendant 12 heures. Après refroidissement dans de la carboglace, le produit attendu est obtenu par distillation à pression atmosphérique.
Point d'ébullition : Eb = 54-55°C

### Stade B : 4-Méthoxybutylcathécholborane

64 mmoles du produit obtenu au stade précédent et 71,4 mmoles de cathécholborane sont chauffées à reflux pendant 18 heures. Le produit attendu est alors obtenu par distillation.
Point d'ébullition : 80°C (p = 0, 1 mmHg)

### Stade C : 4-Aminobutyl boronate de (+)α-pinanediol

20,5 mmoles du produit obtenu au stade précédent sont dissoutes dans 22 ml de tétrahydrofurane anhydre. Après refroidissement à 0°C, 20,5 mmoles de (+)α-pinanediol sont ajoutées au mélange précédent. L'ensemble est laissé 45 minutes à 5-10°C puis 45 minutes à température ambiante. Après évaporation du solvant, 95 ml d'hexane sont ajoutés et l'ensemble est abandonné une nuit au congélateur. Le précipité glacé est alors filtré, lavé par de l'hexane. Ce précipité qui se transforme en huile à température ambiante est alors distillé et conduit au produit attendu.
Point d'ébullition : 80°C (p = 0,01 mmHg)

### Stade D : (R)-1-Chloro-4-méthoxybutyl boronate de (+)α-pinanediol

### Stade E : (R)-1-Amino-4-méthoxybutylboronate de (+)α-pinanediol, chlorhydrate

Les produits décrits dans les stades D et E ont été obtenus selon le procédé décrit par C. KETTNER et coll. (J. Biol. Chem., 265 (30), 18289-18297).

Les abréviations utilisées dans les exemples sont les suivantes :
- Ac: représente acétyl,
- Bz: représente benzyl,
- Suc: représente le groupement succinimido,
- (R)Phe: représente le résidu (R)-phénylalanyl,
- Phi: représente le résidu (2S,3aS,7aS)perhydroindole-2-carbonyl,
- Abo: représente le résidu (3S)-2-azabicyclo[2.2.2]octane-3-carbonyl,
- Ala: représente le résidu alanyl,
- Gly: représente le résidu glycyl,
- Clp: représente le résidu 1-aminocyclopentylcarbonyl de formule : 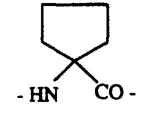
- Asp: représente le résidu aspartyle,
- βAla: représente le résidu β-alanyle,
- Acn: représente le résidu 2-aminocinnamyle de formule :

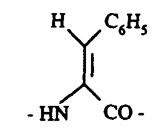

### Exemple 1 : 1-(R)-[(Ac-(R,S)Phe-Phi)amino]-4-aminobutylboronate de (+)α-pinanediol, méthane sulfonate

### Stade A : Ac-(R,S)Phe-Phi-OBz

En utilisant la technique de couplage peptidique (DCC/HOBT) décrite par W.KONIG et R.GEIGER (Ber., 103, 788, 1970) et le diméthylformamide anhydre comme solvant, le produit attendu est préparé à partir de 73 mmoles de Phi-OBz et de Ac-(R)Phe-OH et est purifié par chromatographie sur colonne de silice en utilisant comme éluant un mélange dichlorométhane/éthanol (97/3).
Rendement : 76 %

### Stade B : Ac-(R,S)Phe-Phi-OH

25 mmoles du produit obtenu au stade précédent dans 140 ml d'éthanol sont hydrogénées sous une pression d'hydrogène de 4 kg pendant 12 heures en présence de 2 g de palladium/C à 10 % anhydre. Après filtration du catalyseur, le produit attendu est obtenu après évaporation du solvant.
Rendement : 97 %

### Stade C : Ac-(R,S)Phe-Phi-OSuc

A 4,46 mmoles de N-hydroxysuccinimide dans 50 ml de dichlorométhane anhydre, sont ajoutées 4,46 mmoles du produit obtenu au stade précédent dans 20 ml de dichlorométhane anhydre puis 4,46 mmoles de dicyclohexylcarbodiimide dissous dans du dichlorométhane. L'ensemble est agité 12 heures à température ambiante. Après filtration de la dicyclohexylurée formée, le produit attendu est obtenu après évaporation.

### Stade D : 1-(R)-[(Ac-(R,S)Phe-Phi)amino]-4-bromobutylboronate de (+)α-pinanediol

1 mmole du composé obtenu dans la préparation A dans 10 ml de dichlorométhane anhydre et 1 mmole du composé obtenu au stade précédent sont placées sous atmosphère d'argon à -20°C. 14 ml de triéthylamine sont alors ajoutés goutte à goutte et l'ensemble est maintenu 30 minutes à -20°C. Après retour à température ambiante, le mélange est agité une nuit sous atmosphère d'argon. Après reprise par de l'acétate d'éthyle, lavage à l'eau, au bicarbonate de sodium, à l'eau, à l'acide chlorhydrique 0,2N et enfin à l'eau, la phase organique est séchée et évaporée. Le produit attendu est obtenu après purification sur "sephadex".
Rendement : 90 %

### Stade E : 1-(R)-[(Ac-(R,S)Phe-Phi)amino]4-azidobutylboronate de (+)α-pinanediol

1,5 mmoles du produit obtenu au stade précédent dans 3 ml de diméthylformamide anhydre sont placées à 100°C en présence de 3 mmoles d'azoture de sodium pendant 4 heures. Après 12 heures à température ambiante, l'ensemble est repris par un mélange acétate d'éthyle/eau, la phase organique est lavée plusieurs fois, séchée et évaporée et conduit au produit attendu.
Rendement : 95 %

### Stade F : 1-(R)-[(Ac-(R,S)Phe-Phi)amino]-4-aminobutylboronate de (+)α-pinanediol, méthane sulfonate

1,4 mmoles du composé obtenu au stade précédent dans 25 ml de méthanol anhydre sont hydrogénées en présence de 1,4 mmoles d'acide méthane sulfonique en utilisant 50 mg de palladium/C à 10 % comme catalyseur, pendant 2 heures. Après filtration du catalyseur, rinçage et évaporation, on obtient le produit attendu.
Rendement : 95 %

### Exemple 2 : 1-(R)-[(Ac-(R,S)Phe-Phi)amino]-4-guanidinobutylboronate de (+)α-pinanediol, méthane sulfonate

1,3 mmoles du composé obtenu dans l'exemple 1 et 10,3 mmoles de cyanamide sont portées à reflux dans 8 ml d'éthanol anhydre pendant 10 jours. Après évaporation de l'éthanol, passage sur résine sephadex en reprenant le résidu par du méthanol, on obtient le produit attendu.
Rendement : 91 %

### Exemple 3 : Acide 1-(R)-[(Ac-(R,S)Phe-Phi)amino]-4-guanidinobutyl boronique, acétate

1,2 mmoles du composé obtenu dans l'exemple 2 dans 30 ml de dichlorométhane anhydre sont refroidies à -78°C sous atmosphère d'argon. 4,8 mmoles de trichlorure de bore sont alors additionnées goutte à goutte en 30 minutes. La température est amenée à 0°C et l'ensemble agité 30 minutes. 10 ml d'eau glacée sont alors ajoutés et après 15 minutes d'agitation, le mélange est amené à température ambiante. La phase organique est décantée, extraite par 10 ml d'un mélange eau/acide acétique (90/10). La phase aqueuse restante est lavée à l'éther et les phases aqueuses réunies sont évaporées. Le résidu est purifié sur Bio-gel en utilisant comme éluant un mélange eau/acide acétique (90/10) et conduit au produit attendu qui est lyophilisé.

| Microanalyse élémentaire : | | | |
|---|---|---|---|
| | C % | H % | N % |
| calculé | 56,45 | 7,54 | 14,63 |
| trouvé | 54,71 | 7,26 | 13,59 |

### Exemple 4 : Acide 1-(R)-[(Ac-(R)Phe-Phi)amino]-4-aminobutyl boronique, acétate

Ce composé a été obtenu selon le même procédé que celui décrit pour l'exemple 3 en utilisant comme produit de départ le composé de l'exemple 1.

### Exemple 5 : (R)-[(Ac-(R)Phe-Phi)amino]-(cyclopentyl)méthylboronate de (+)α-pinanediol

### Stades A à C : ces stades sont identiques aux stades A à C de l'exemple 1.

### Stade D :

Ce composé a été obtenu selon le procédé décrit au stade D de l'exemple 1 à partir du composé obtenu au stade C et du composé obtenu dans la préparation B.
Rendement : 90 %

### Exemple 6 : Acide (R)-[(Ac-(R)Phe-Phi)amino]-(cyclopentyl)méthyl boronique, acétate

Ce composé a été obtenu selon le procédé décrit pour l'exemple 3 en utilisant comme produit de départ le composé de l'exemple 5.

Les composés des exemples 7 à 14 ont été synthétisés selon le même procédé que celui décrit pour l'exemple 1 en utilisant les produits de départ correspondants.

### Exemple 7 : 1-(R)-[(Ac-(R,S)Phe-Abo)amino]-4-aminobutylboronate de (+)α-pinanediol, méthane sulfonate

### Exemple 8 : 1-(R)-{[Ac-(R)-(cyclohexyl)Ala-(N-cyclopentyl)Gly]amino}-4-aminobutylboronate de (+)α-pinanediol, méthane sulfonate

### Exemple 9 : 1-(R)-{[Ac-(R)Phe-(N-cyclobutyl)Gly]amino}-4-aminobutylboronate de (+)α-pinanediol, méthane sulfonate

### Exemple 10 : 1-(R)-{[Ac-(R,S)Phe-(N-cyclohexyl)Gly]amino}-4-aminobutylboronate de (+)α-pinanediol, méthane sulfonate

### Exemple 11 : 1-(R)-{[Ac-(R,S)Phe-(N-cyclopentyl)Gly]amino}-4-aminobutylboronate de (+)α-pinanediol, méthane sulfonate

### Exemple 12 : 1-(R)-{[Ac-(R)Phe-(N-cyclopentyl)Gly]amino}-4-aminobutylboronate de (+)α-pinanediol, méthane sulfonate

### Exemple 13 : 1-(R)-{[Ac-(S)Phe-(N-cyclopentyl)Gly]amino}-4-aminobutylboronate de (+)α-pinanediol, méthane sulfonate

### Exemple 14 : 1-(R)-{[Ac-(R,S)Phe-(N-(R,S)indan-1-yl)Gly]amino}-4-aminobutylboronate de (+)α-pinanediol, méthane sulfonate

Les exemples 15 à 22 ont été synthétisés selon le même procédé que celui décrit pour l'exemple 2 en utilisant les produits de départ correspondants.

### Exemple 15 : 1-(R)-[(Ac-(R,S)Phe-Abo)amino]-4-guanidinobutylboronatede (+)α-pinanediol, benzène sulfonate

A partir du composé décrit dans l'exemple 7.

### Exemple 16 : 1-(R)-{[Ac-(R)-(cyclohexyl)Ala-(N-cyclopentyl)Gly]amino}-4-guanidinobutylboronate de (+)α-pinanediol, benzène sulfonate

A partir du composé de l'exemple 8.

### Exemple 17 : 1-(R)-{[Ac-(R)Phe-(N-cyclobutyl)Gly]amino}-4-guanidinobutylboronate de (+)α-pinanediol, benzène sulfonate

A partir du composé de l'exemple 9.

### Exemple 18 : 1-(R)-{[Ac-(R,S)Phe-(N-cyclohexyl)Gly]amino}-4-guanidinobutylboronate de (+)α-pinanediol, benzène sulfonate

A partir du composé de l'exemple 10.
Spectre de masse : FAB⁺ : [M+H] : m/z = 636

### Exemple 19 : 1-(R)-{[Ac-(R,S)Phe-(N-cyclopentyl)Gly]amino}-4-guanidinobutylboronate de (+)α-pinanediol, benzène sulfonate

A partir du composé de l'exemple 11.
Spectre de masse : FAB⁺ : [M+H] : m/z = 622

### Exemple 20 : 1-(R)-{[Ac-(R)Phe-(N-cyclopentyl)Gly]amino}-4-guanidinobutylboronate de (+)a-pinanediol, trifluoroacétate

A partir du composé de l'exemple 12.
Spectre de masse : FAB⁺ : [M+H] : m/z = 622

### Exemple 21 : 1-(R)-{[Ac-(S)Phe-(N-cyclopentyl)Gly]amino}-4-guanidinobutylboronate de (+)α-pinanediol, benzène sulfonate

A partir du composé de l'exemple 13.

### Exemple 22 : 1-(R)-{[Ac-(R,S)Phe-(N-(R,S)indan-1-yl)Gly]amino}-4-guanidinobutylboronate de (+)α-pinanediol, benzène sulfonate

A partir du composé de l'exemple 14.

Les exemples 23 à 30 ont été synthétisés selon le même procédé que celui décrit dans l'exemple 3 à partir des produits de départ correspondants.

### Exemple 23 : Acide 1-(R)[(Ac-(R,S)Phe-Abo)amino]-4-guanidinobutyl boronique, acétate

A partir du composé de l'exemple 15.
Spectre de masse : FAB⁺ : [M+glycérol-2H₂O+H]⁺ m/z = 557

### Exemple 24 : Acide 1-(R)-{[Ac-(R)-(cyclohexyl)Ala-(N-cyclopentyl)Gly]amino}-4-guanidinobutyl boronique, acétate

A partir du composé de l'exemple 16.
Spectre de masse : FAB⁺ : [M+glycérol-2H₂O+H]⁺ m/z = 586

### Exemple 25 : Acide 1-(R)-{[Ac-(R)Phe-(N-cyclobutyl)Gly]amino}-4-guanidinobutyl boronique, acétate

A partir du composé de l'exemple 17.
Spectre de masse : FAB⁺ : [M+glycérol-2H₂O+H]⁺ m/z= 566

### Exemple 26 : Acide 1-(R)-{[Ac-(R,S)Phe-(N-cyclohexyl)Gly]amino}-4-guanidinobutyl boronique, méthane sulfonate

A partir du composé de l'exemple 18.

| Microanalyse élémentaire : | | | |
|---|---|---|---|
| | C % | H % | N % |
| calculé | 55,52 | 7,71 | 14,94 |
| trouvé | 56,20 | 7,25 | 14,54 |

### Exemple 27 : Acide 1-(R)-{[Ac-(R,S)Phe-(N-cyclopentyl)Gly]amino}-4-guanidinobutyl boronique, acétate

A partir du composé de l'exemple 19.
Spectre de masse : FAB⁺ : [M+glycérol-2H₂O+H]⁺ m/z = 580

### Exemple 28 : Acide 1-(R)-{[Ac-(R)Phe-(N-cyclopentyl)Gly]amino}-4-guanidinobutyl boronique, acétate

A partir du composé de l'exemple 20.
Spectre de masse : FAB⁺ : [M+glycérol-2H₂O+H]⁺ m/z = 580

### Exemple 29 : Acide 1-(R)-{[Ac-(S)Phe-(N-cyclopentyl)Gly]amino}-4-guanidinobutyl boronique, acétate

A partir du composé de l'exemple 21.
Spectre de masse : FAB⁺ : [M+glycérol-2H₂O+H]⁺ m/z = 580

### Exemple 30 : Acide 1-(R)-{[Ac-(R,S)Phe-(N-(R,S)indan-1-yl)Gly]amino}-4-guanidinobutyl boronique, acétate

A partir du composé de l'exemple 22.
Spectre de masse : FAB⁺ : [M+glycérol-2H₂O+H]⁺ m/z = 593

### Exemple 31 : 1-(R)-[(Ac-(R,S)Phe-Phi)amino]4-méthoxybutylboronate de (+)α-pinanediol

Ce composé a été synthétisé selon le procédé décrit dans l'exemple 1 (stades A à D) en remplacant au stade D le composé obtenu dans la préparation A par le composé obtenu dans la préparation C.

| Microanalyse élémentaire : | | | |
|---|---|---|---|
| | C % | H % | N % |
| calculé | 67,63 | 8,43 | 6,76 |
| trouvé | 67,16 | 8,39 | 7,10 |

### Exemple 32 : Acide 1-(R)-[(Ac-(R,S)Phe-Phi)amino]-4-méthoxy boronique

Ce composé a été synthétisé selon le procédé décrit dans l'exemple 3 à partir du composé décrit dans l'exemple 31.
Spectre de masse : FAB⁺ : [M+glycérol-H₂O+H]⁺ m/z = 543

### Exemple 33 : 1-(R)-{[N-(5-Diméthylamino-1-naphtalènesulfonyl)Gly-Phi]amino}4-aminobutylboronate de (+)α-pinanediol, benzène sulfonate

### Stades A à D :

Ces stades A à D sont identiques aux stades A à D de l'exemple 1 mais au stade A on remplace Ac-(R)Phe-OH par Boc-Gly-OH.

### Stade E : 1-(R)-[(Gly-Phi)amino]-4-bromobutylboronate de (+)α-pinanediol, trifluoroacétate

6,64 mmoles du produit obtenu au stade précédent en solution du 20 ml de dichlorométhane à 0°C sont déprotégées en présence de 53 mmoles d'acide trifluoroacétique. Après 1 heure d'agitation, 24 heures à température ambiante, évaporation du solvant et séchage, on obtient le produit attendu.

### Stade F : 1-(R)-{[N-(5-Diméthylamino-1-naphtalènesulfonyl)Gly-Phi]amino}-4-bromobutylboronate de (+)α-pinanediol

A 6,53 mmoles du composé obtenu au stade précédent en solution dans 100 ml de tétrahydrofurane, à l'abri de la lumière, sont ajoutées 19,8 mmoles de triéthylamine. Après 5 minutes d'agitation, 6,6 mmoles de chlorure de Dansyl sont ajoutées par portions. Après 4 heures d'agitation, évaporation du solvant, reprise du résidu par de l'acétate d'éthyle, lavage par de l'hydrogénocarbonate de sodium et évaporation on obtient le produit attendu après purification sur colonne SEPHADEX LH 20 en utiliant le méthanol comme éluant.

### Stades G et H :

Ces stades sont identiques aux stades E et F de l'exemple 1 mais au stade F l'acide méthane sulfonique est remplacé par l'acide benzène sulfonique.

### Exemple 34 : 1-(R)-{[N-(5-Diméthylamino-1-naphtalènesulfonyl)Gly-Phi]amino}-4-guanidinobutylboronate de (+)α-pinanediol, benzène sulfonate

Le produit attendu est obtenu selon le procédé décrit dans l'exemple 2 à partir du composé de l'exemple 33.
Spectre de masse : FAB⁺ (I⁺) : [M+H]⁺ m/z = 750

### Exemple 35 : Acide 1-(R)-{[N-(5-Diméthylamino-1-naphtalènesulfonyl)Gly-Phi]amino}-4-guanidinobutyl boronique, diacétate

Le produit attendu est obtenu selon le procédé décrit dans l'exemple 3 à partir du composé de l'exemple 34.
Spectre de masse : FAB⁺ (I⁺) : [M+H-glycérol-H₂O]⁺ m/z = 672

Les exemples 36 à 61 ont été synthétisés selon le procédé décrit dans l'exemple 3 en utilisant les produits de départ correspondants.

### Exemple 36 : Acide 1-(R)-{[Ac-(R)Phe-(N-cyclopenta-2,4-diènyl)Gly]amino}-4-guanidinobutylboronique, acétate

### Exemple 37 : Acide 1-(R)-{[Ac-(R)Phe-(N-bicyclo[2.1.1]hex-5-yl)Gly]amino}-4-guanidinobutylboronique, acétate

### Exemple 38 : Acide 1-(R)-{[Ac-(R)Phe-(N-1,2-oxazolidin-3-yl)Gly]amino}4-guanidinobutylboronique, acétate

### Exemple 39 : Acide 1-(R)-{[Ac-(R)Phe-(N-cyclopent-3-ènyl)Gly]amino}-4-guanidinobutylboronique, acétate

### Exemple 40 : Acide 1-(R)-{[Ac-(R)Phe-(N-cyclopent-2-ènyl)Gly]amino}-4-guanidinobutylboronique, acétate

### Exemple 41 : Acide 1-(R)-{[Ac-(R)Phe-(N-(1-méthyl)cyclopentyl)Gly]amino}-4-guanidinobutylboronique, acétate

### Exemple 42 : Acide 1-(R)-{[Ac-(R)Phe-(N-(3,5-diméthyl)cyclopentyl)Gly]amino}-4-guanidinobutylboronique, acétate

### Exemple 43 : Acide 1-(R)-{[Ac-(R)Phe-(N-(2,2-diméthyl)cyclopentyl)Gly]amino}-4-guanidinobutylboronique, acétate

### Exemple 44 : Acide 1-(R)-{[Ac-(R)Phe-(N-(1-cyclopentyl)2,2,2-trifluoroéthyl)Gly]amino}-4-guanidinobutylboronique, acétate

### Exemple 45 : Acide 1-(R)-{[Ac-(R)Phe-(N-cyclopropyl)Gly]amino}-4-guanidinobutylboronique, acétate

### Exemple 46 : Acide 1-(R)-{[Ac-(R)Phe-(N-(3-méthyl)cyclobutyl)Gly]amino}-4-guanidinobutylboronique, acétate

### Exemple 47 : Acide 1-(R)-{[Ac-(R)Phe-(N-(1-éthyl)propyl)Gly]amino}-4-guanidinobutylboronique, acétate

### Exemple 48 : Acide 1-(R)-{[Ac-(R)(3-thiényl)Ala-(N-cyclopentyl)Gly]amino}-4-guanidinobutylboronique, acétate

### Exemple 49 : Acide 1-(R)-{[Ac-(R)(diphényl)Ala-(N-cyclopentyl)Gly]amino}-4-guanidinobutylboronique, acétate

### Exemple 50 : Acide 1-(R)-{[(R)Phe-(N-cyclopentyl)Gly]amino}-4-guanidinobutylboronique, acétate

### Exemple 51 : Acide 1-(R)-{[(N-méthyl)(R)Phe-(N-cyclopentyl)Gly]amino}-4-guanidinobutylboronique, acétate

### Exemple 52 : Acide 1-(R)-{[Ac-(R)(benzocyclobutyl)Gly-(N-cyclopentyl)Gly]amino}-4-guanidinobutylboronique, acétate

### Exemple 53 : Acide 1-(R)-{[(N-méthyl)-(R)-phénylGly-(N-cyclopentyl)Gly]amino}-4-guanidinobutylboronique, acétate

### Exemple 54 : Acide 1-(R)-{[(N-méthoxycarbonylméthyl)-(R)Phe-(N-cyclopentyl)Gly]amino}-4-guanidinobutylboronique, acétate

### Exemple 55 : Acide 1-(R)-{[Ac-[di(phénylméthyl)]Gly-(N-cyclopentyl)Gly]amino}-4-guanidinobutylboronique, acétate

### Exemple 56 : Acide 1-(R)-{[Ac-(R)Phe-(cyclopentyl)Gly]amino}-4-guanidinobutylboronique, acétate

### Exemple 57 : Acide 1-(R)-{[Ac-(R)Phe-Clp]-4-guanidinobutylboronique, acétate

### Exemple 58 : Acide 1-(R)-{[Ac-(R)Phe-(N-cyclopentyl)Ala]amino}-4-guanidinobutylboronique, acétate

### Exemple 59 : Acide 1-(R)-{[Ac-(R)Phe-(N-cyclopentyl)Asp]amino}-4-guanidinobutylboronique, acétate

### Exemple 60 : Acide 1-(R)-{[Ac-(R)Phe-(N-cyclopentyl)-β-Ala]amino}-4-guanidinobutylboronique, acétate

### Exemple 61 : Acide 1-(R)-{[Ac-(ACN)-(N-cyclopentyl)Gly]amino}-4-guanidinobutylboronique, acétate

### Etude pharmacologique des composés de l'invention

### Exemple 62 : Activité antithrombotique

Le modèle de thrombose expérimentale utilisé est celui d'une thrombose veineuse induite chez le rat Wistar mâle par ligature de la veine cave inférieure sous la veine rénale gauche.

Les animaux à tester sont anesthésiés par administration intrapéritonéale de Brietal® (méthohexital sodique) à la dose de 1 mg/kg.

La ligature est alors réalisée. Le produit à tester est injecté 15 minutes avant la ligature par voie intraveineuse. 25 minutes après la ligature, la veine est excisée et le caillot est récupéré puis pesé après passage dans une étuve à 37°C pendant 12 heures.

Les composés de l'invention ont été testés aux doses de 0,1, 0,25 et 0,5 mg/kg et comparés au DUP 714 utilisé comme composé témoin.

Les résultats de ce test ont montré que les composés de l'invention permettent une diminution significative du poids du caillot aussi importante que celle observée lors de l'injection du composé de référence (DUP 714). A 0,25 mg/kg, les composés de l'invention inhibent la formation du caillot d'environ 50 %.

### Exemple 63 : Activité anti-coagulante, mesure du temps de thrombine,chez le rat et chez l'homme

En présence d'une quantité standard de thrombine, un plasma normal coagule en un temps défini et constant, appelé temps de thrombine (TT).

Tout allongement de ce temps traduit une anomalie de la fibrinoformation (coagulation).

Les rats Sprague-Dawley sont anesthésiés avec du pentobarbital sodique (60 mg/kg, i.p.), l'artère carotide est cathétérisée et les prélèvements sanguins réalisés dans une solution de citrate trisodique (0,109 M). Un plasma pauvre en plaquettes est obtenu par centrifugation des échantillons sanguins (3000 g, 15 minutes).

Des prélèvements de sang veineux au pli du coude sont également réalisés chez l'homme. L'anticoagulation des prélèvements et la préparation du plasma pauvre en plaquettes sont identiques à celles réalisées avec le sang de rat.

Le temps de thrombine est réalisé avec le réactif thrombine Prest et déterminé automatiquement en utilisant un coagulomètre.

L'antagoniste ou le solvant (10 µl) est additionné au plasma (90 µl), puis incubé 2 minutes à 37°C. 100 µl de thrombine sont ajoutés en déclenchant le chronomètre.

Dans ces conditions, les TT obtenus dans le plasma témoin sont de l'ordre de 30 secondes chez le rat et de 20 secondes chez l'homme. L'activité d'un antagoniste est évaluée par sa capacité à prolonger ce temps de thrombine par rapport au témoin.

Dans ces conditions, les composés de l'invention permettent une prolongation du temps de thrombine de 50 fois et plus. L'effet des inhibiteurs est mesuré et la concentration qui multiplie par 2 le temps de thrombine (Ctt₂) est déterminée. Les résultats sont reproduits dans le tableau suivant :

| Produits | Rat CTT₂ (µM) | HOMME CTT2 (µM) |
|---|---|---|
| ex. 3 | 0.23 | 0.07 |
| ex. 23 | 0.17 | 0.11 |
| ex. 27 | - | 0.20 |
| ex. 28 | - | 0.19 |
| Réf. : DUP 714 | 0.32 | 0.18 |

Ainsi avec le composé de l'exemple 23, la concentration nécessaire pour doubler le temps de thrombine chez le rat est 2 fois inférieure à celle obtenue avec le composé de référence (DUP 714). Avec le composé de l'exemple 3, la concentration nécessaire pour doubler le temps de thrombine chez l'homme est 2,5 fois inférieure à celle obtenue avec le composé de référence (DUP 714).

### Exemple 64 : Inhibition de la thrombine et des sérines protéases de la coagulation et de la fibrinolyse

Pour évaluer in vitro l'activité inhibitrice des produits boroArginiques sur la thrombine humaine (Sigma, activité spécifique 3230 UNIH/mg), le fibrinogène humain purifié (4 mM, stago) (Fg) ou le substrat chromogène H-D-Phe-Pip-Arg-pNa (44 mM, S2238, Kabi) ont été ajoutés à une quantité donnée de thrombine (0,7 ou 2 nM) préalablement incubée avec ou sans l'inhibiteur à tester (20°C, 10 minutes).

Pour évaluer in vitro la sélectivité de ces produits vis-à-vis de différentes sérines protéases de la fibrinolyse et de la coagulation, le même protocole a été appliqué à la plasmine humaine purifiée (2 nM, Stago), à la protéine C activée humaine purifiée (2 nM, STAGO), au facteur X activé humain purifié (2 nM, Stago), à l'activateur tissulaire du plasminogène (2 nM, Calbiochem), à l'urokinase humaine purifiée (2 nM, Sigma), à la kallikreine plasmatique humaine purifiée (2 nM, Calbiochem) en utilisant pour substrats différents peptides paranitroanilidés : <Glu-Phe-Lys-pNA (0.50 mM, S 2403, Kabi) pour la plasmine, N_Cbo-Arg-Gly-Arg-pNA (0.38 mM, S 2765, Kabi) pour le facteur Xa, <Glu-Pro-Arg-pNa (0,52 mM, S 2366, Kabi) pour la protéine C activée, H-D-Pro-Phe-Arg-pNA (0.45 mM, S 2302, Kabi) pour la kallikreine, H-D-Ile-Pro-Arg-pNA (0.48 mM, S 2288, Kabi), <Glu-Gly-Arg-pNA (0.56 mM, S 2444, Kabi).

Inhibiteurs, enzymes et substrats sont dilués dans le même tampon (tampon phosphate 0,01 mM, pH 7,4, contenant 0,12 M de chlorure de sodium et 0,05 % de sérum albumine bovine) puis distribués dans une microplaque en polystyrène sous un volume de 50 µl.

La fibrine formée par la thrombine ou par le paranitroanilide libéré par l'action de la sérine protéase est mesurée spectrophotométriquement à 405 nm après 15 à 30 minutes de réaction à 20°C.

Le tableau ci-dessous donne la concentration de composé inhibant 50 % de l'activité enzymatique (CI 50) en présence du produit de référence (DUP 714) et des composés des l'exemples 25, 27, 28 par rapport au contrôle sans produit. Les résultats démontrent que les composés des exemples 25 et 28 sont des inhibiteurs de la thrombine humaine plus puissants que le composé de référence vis-à-vis du fibrinogène humain. Les composés des exemples 25, 27 et 28 possédent une sélectivité supérieure vis-à-vis des sérines protéases de la coagulation et de la fibrinolyse, à celle du DUP 714.

| **CI50 (nM)** | | | | | | | | |
|---|---|---|---|---|---|---|---|---|
| Exemples | Thrombine | | | | | | | |
| | Fg | S2238 | Plasmine | tPA | Urokinase | FXa | Kalli | PCa |
| Ref. DUP 714 | 0.55 | 2.0 | 28 | 8 | 24 | 41 | 8 | 17 |
| Ex. 28 | 0.46 | 2.0 | 445 | 47 | 1821 | 225 | 91 | 82 |
| Ex. 25 | 0.45 | 2.0 | 818 | 14 | 1636 | 356 | 75 | 211 |
| Ex. 27 | 0.79 | 2.5 | 735 | 38 | 3176 | 1757 | 104 | 149 |

### Exemple 65 : Mesure de l'activité anti-coagulante ex vivo. Administration des produits per os (Rat éveillé).

- Les rats OFA, à jeun, sont anesthésiés à l'éther. L'artère caudale est dégagée et cathétérisée. Le cathéter est purgé avec du sérum physiologique citraté (1/40). Après installation du cathéter, l'animal est placé dans une cage à contention et 1,5 cm³ de sang artériel est prélevé sur citrate 0,109 M (1/9).
- 1 heure après le réveil de l'animal, les produits à tester sont administrés per os à une dose unique de 10 mg/kg.
- Des prélèvements artériels (1,5 ml) sont alors effectués à 30 minutes, 60 minutes, 2 heures.
- A chaque prélèvement, 1,5 ml de sérum physiologique est réinjecté à l'animal.
- Les tubes de sang sont centrifugés 15 minutes à 3000 g pour la préparation du plasma. Le temps d'apparition du phénomène de coagulation est mesuré après addition de thrombine Prest.
- Les composés de l'invention augmentent de façon dose-dépendante le temps de thrombine (TT) et leur activité est égale ou supérieure à celle obtenue avec le composé de référence (DUP 714) durant les 2 premières heures. Les résultats sont reproduits dans le tableau suivant et montrent les indices d'augmentation du temps de thrombine.

| | Temps (heures) | | |
|---|---|---|---|
| Produit | 0.5 | 1 | 2 |
| DUP 714 | 4.0 | 3.7 | 3.9 |
| Ex. 3 | 7.7 | 7.1 | 5.6 |

### Exemple 66 : Mesure de l'activité anti-coagulante ex vivo. Administration des produits par voie intraveineuse (i.v.)

Les rats OFA, à jeun ou non, sont anesthésiés au pentobarbital (60 mg/kg, i.p.). L'artère carotide et la veine jugulaire sont dégagées et cathétérisées. Les cathéters sont purgés avec du sérum physiologique citraté (1/40). Après installation des cathéters, un prélèvement de 1,5 cm³ de sang artériel est effectué sur citrate 0,109 M (1/9).
- 30 minutes après, le produit à tester est administré sous un volume de 1 ml en i.v.
- Des prélèvements artériels (1,5 ml) sont alors effectués à 1 minute 30, 5, 15, 30 et 60 minutes.
- A chaque prélèvement, 1,5 ml de sérum physiologique citraté est réinjecté à l'animal via la carotide.
- Les tubes de sang sont centrifugés 15 minutes à 3000 g (préparation de plasma).
- 100 µl de plasma sont incubés avec 100 µl de céphaline activée. Le temps d'apparition du phénomène de coagulation est mesuré, après addition de 100 µl de calcium.
- Les composés de l'invention, testés à la dose de 0,5 mg/kg augmentent de façon dose-dépendante le temps de céphaline activée (TCA). L'effet est comparable ou plus important que celui du composé de référence (DUP 714). L'augmentation du TCA notée avec les composés de l'invention est toujours significative 60 minutes après leur administration. Les résultats sont reproduits dans le tableau suivant et montrent les indices d'augmentation du temps de coagulation.

| | Temps (minutes) | | | | |
|---|---|---|---|---|---|
| Produit | 15.5 | 5 | 15 | 30 | 60 |
| DUP 714 | 7.6 | 3.4 | 2.0 | 1.7 | 1.4 |
| Ex. 3 | 7.3 | 4.7 | 3.6 | 2.6 | 2.0 |
| Ex. 27 | 5 | 2.3 | 1.7 | 1.5 | 1.3 |

### Exemple 67 : Activité anti-coagulante et thrombopénie p.o. chez le chien

Les produits ont été administrés par voie orale chez le chien.2 heures et 4 heures après le traitement, le sang a été prélevé par voie intraveineuse. Le plasma est préparé, les plaquettes sont comptées et le test de temps de thrombine est pratiqué.
Le tableau démontre que le DUP 714 à 2.5 mg/kg a augmenté le TT mais a provoqué une forte thrombopénie. Les produits de l'invention à 5 mg/kg ont provoqué des augmentations du TT plus importantes que le DUP 714 sans modification du nombre de plaquettes.

| | | Indice d'augmentation TT | Variation du nbre de plaquettes (% de contrôle) |
|---|---|---|---|
| Ref. DUP 714 | 2 hr | 7 | - 32 % |
| (2.5 mg/kg) | 4 hr | 2 | - 16 % |
| Ex. 27 | 2 hr | 11 | - 4 % |
| (5 mg/kg) | 4 hr | 3 | - 1 % |
| Ex. 28 | 2 hr | 11 | - 4 % |
| (5 mg/kg) | 4 hr | 2 | + 1 % |

### Exemple 68 : Composition pharmaceutique

| Formule de préparation pour 1000 comprimés dosés à 10 mg : | |
|---|---|
| Composé de l'exemple 3 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 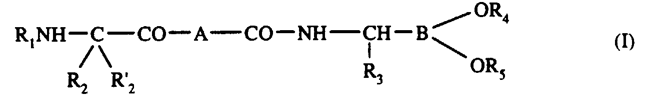 dans laquelle :
R₁ représente un atome d'hydrogène ou un groupement acyle (C₁-C₆) linéaire ou ramifié, alkyle (C₁-C₆) linéaire ou ramifié, benzyle, alkoxycarbonyle (C₁-C₆) linéaire ou ramifié, benzyloxycarbonyle, phénoxycarbonyle, 5-[(diméthyl)amino]naphtylsulfonyl, alkoxycarbonylméthyle ou carboxyméthyle,
R₂ représente un atome d'hydrogène ou un groupement :
- phényle,
- benzyle (substitué ou non sur le noyau phényle par un ou plusieurs atomes d'halogène ou groupements alkyle (C₁-C₆) linéaire ou ramifié, alkoxy (C₁-C₆) linéaire ou ramifié, hydroxy ou bicyclooct-1-yle (substitué ou non par un groupement hydroxy ou alkoxy (C₁-C₆) linéaire ou ramifié)),
- thiénylméthyle,
- (pyridinyl)méthyle,
- diphénylméthyle,
- fluorényle,
- naphtylméthyle,
- benzocyclobutyle,
- (dicyclopropylméthyl)méthyle,
- indanyle,
- ou, cycloalkyl (C₃-C₇)méthyle,
R'₂ représente un atome d'hydrogène ou un groupement benzyle
ou bien
R₂ et R'₂ représentent ensemble C₆H₅-CH=,
R₃ représente un groupement :
- alkyle (C₂-C₄) linéaire ou ramifié substitué par un groupement alkoxy (C₁-C₆) linéaire ou ramifié, phénoxy, guanidino, amidino, amino, 2-tétrahydrofuryle ou cycloalkyle (C₃-C₇),
- guanidinophényle, amidinophényle, aminophényle,
- guanidinobenzyle, amidinobenzyle, aminobenzyle,
- ou, cycloalkyle (C₃-C₇),
R₄ et R₅ représentent chacun un atome d'hydrogène ou un groupement alkyle (C₁-C₆) linéaire ou ramifié,
ou 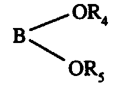 forme un ester boronique de pinanediol,
A représente l'un quelconque des groupements suivants :
* 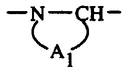
dans lequel :
A₁ représente avec les atomes d'azote et de carbone avec lesquels il est lié une structure cyclique choisie parmi les structures suivantes :
- perhydroindole,
- 2-azabicyclo[2.2.2]octane,
- 2-azabicyclo[3.3.0]octane,
- 2-azabicyclo[2.2.1]heptane,
- perhydroisoindole,
- indoline,
- isoindoline,
- perhydroquinoléine,
- perhydroisoquinoléine,
- 1,2,3,4-tétrahydroquinoléine,
- 1,2,3,4-tétrahydroisoquinoléine,
- thiazolidine éventuellement substituée par un ou plusieurs groupements alkyl (C₁-C₆) linéaire ou ramifié,
- 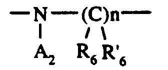
dans lequel:
n représente 1 ou 2,
R₆ représente un atome d'hydrogène ou un groupement cycloalkyle (C₃-C₇), alkyle (C₁-C₆) linéaire ou ramifié ou carboxyméthyle,
R'₆ représente un atome d'hydrogène,
ou bien
R₆ et R'₆ forment avec l'atome de carbone qui les portent un groupement cycloalkyle (C₃-C₇),
A₂ représente un groupement alkyle (C₁-C₆) linéaire ou ramifié, phényle, indanyle, cycloalkyle (C₃-C₇) (substitué ou non par un ou plusieurs groupements méthyle) cycloalkényle (C₃-C₇), cyclopentadiènyle, 2,2,2,-trifluoro-1-cyclopentyléthyl, bicyclo[2.1.1]hexyl, bicyclo[2.2.1]heptyl ou un groupement : 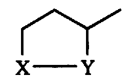 dans lequel
X et Y différents représentent un atome d'oxygène, de soufre, un groupement NH ou CH₂-,
ou bien
A₂ représente un atome d'hydrogène (à la condition que dans ce cas R₆ représente un groupement cycloalkyle (C₃-C₇) et R'₆ représente un atome d'hydrogène, ou bien R₆ et R'₆ forment avec l'atome de carbone qui les portent un groupement cycloalkyle (C₃-C₇)),
leurs énantiomères, diastéréoisomères et épimères ainsi que leurs sels d'addition à un acide ou à une base pharmaceutiquement acceptable.

2. Composés de formule (I) selon la revendication 1 dans laquelle R₁ représente un groupement acétyl, leurs énantiomères, diastéréoisomères et épimères ainsi que leurs sels d'addition à un acide ou à une base pharmaceutiquement acceptable.

3. Composés de formule (I) selon la revendication 1 dans laquelle R₂ représente un groupement benzyl, leurs énantiomères, diastéréoisomères et épimères ainsi que leurs sels d'addition à un acide ou à une base pharmaceutiquement acceptable.

4. Composés de formule (I) selon la revendication 1 dans laquelle A représente un groupement 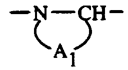 tels que défini dans la revendication 1, leurs énantiomères, distéréoisomères et épimères ainsi que leurs sels d'addition à un acide ou à une base pharmaceutiquement acceptable.

5. Composés de formule (I) selon la revendication 1 dans laquelle A représente un groupement 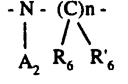 tels que défini dans la revendication 1, leurs énantiomères, diastéréoisomères et épimères ainsi que leurs sels d'addition à un acide ou à une base pharmaceutiquement acceptable.

6. Composés de formule (I) selon la revendication 4 dans laquelle A₁ représente avec les atomes d'azote et de carbone avec lesquels il est lié un cycle perhydroindole, leurs énantiomères, diastéréoisomères et épimères ainsi que leurs sels d'addition à un acide ou à une base pharmaceutiquement acceptable.

7. Composés de formule (I) selon la revendication 1 dans laquelle R₃ représente un groupement guanidinoalkyle (C₂-C₄) linéaire ou ramifié, leurs énantiomères, diastéréoisomères et épimères ainsi que leurs sels d'addition à un acide ou à une base pharmaceutiquement acceptable.

8. Composés de formule (I) selon la revendication 1 dans laquelle R₃ représente un groupement cycloalkyle (C₃-C₇), leurs énantiomères, diastéréoisomères et épimères ainsi que leurs sels d'addition à un acide ou à une base pharmaceutiquement acceptable.

9. Composé de formule (I) selon la revendication 1 qui est l'acide 1-(R)-[(Ac-(R)Phe-Phi)amino]-4-guanidinobutyl boronique, Ac représentant acétyl, (R)Phe représentant (R)-phénylalanyl, Phi représentant (2S,3aS,7aS) perhydroindole-2-carbonyl, ses isomères ainsi que ses sels d'addition à un acide ou à une base pharmaceutiquement acceptable.

10. Composé de formule (I) selon la revendication 1 qui est Acide 1-(R)-{[Ac-Phe-(N-cyclopentyl)Gly]amino)-4-guanidinobutyl boronique, acétate, Ac représentant acétyl, Phe représentant phénylalanyl de configuration (R,S) ou (R), Gly représentant glycyl, ses énantiomères ainsi que ses sels d'addition à un acide ou à une base pharmaceutiquement acceptable.

11. Procédé de préparation des composés de formule (I) selon la revendication 1, caractérisé en ce que l'on fait réagir un amino-acide protégé de formule (II), dont on a éventuellement séparé les isomères par une technique classique de séparation : 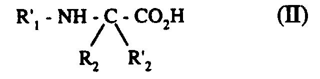 dans laquelle :
R'₁ représente un groupement acyle (C₁-C₆) linéaire ou ramifié, benzyle, alkoxycarbonyle (C₁-C₆) linéaire ou ramifié, benzyloxycarbonyle ou phénoxycarbonyle,
et
R₂ et R'₂ ont la même signification que dans la formule (I),
que l'on fait réagir selon une technique de couplage peptidique avec un deuxième amino-acide protégé de formule (III) dont on a éventuellement séparé les isomères selon une technique classique de séparation,
HA-CO₂CH₂C₆H₅ (III)
dans laquelle A a la même signification que dans la formule (I), pour conduire au composé de formule (IV) : 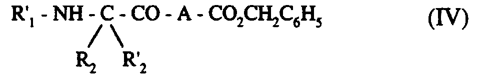 dans laquelle R'₁, R₂, R'₂ et A ont la même signification que précédemment,
dont on déprotège la fonction acide par hydrogénation catalytique pour conduire au composé de formule (V) : 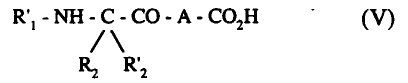 dans laquelle R'₁, R₂, R'₂ et A ont la même signification que précédemment,
que l'on met en réaction avec du N-hydroxysuccinimide en présence de 1,3-dicyclohexylcarbodiimide en milieu anhydre, pour conduire au composé de formule (VI) : 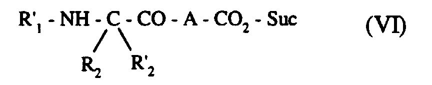 dans laquelle R'₁, R₂, R'₂ et A ont la même signification que précédemment et Suc représente un radical succinimido,
que l'on met en réaction en milieu basique avec un composé de formule (VII) : 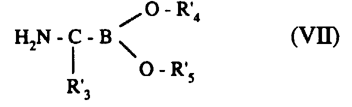 dans laquelle :
R'₃ représente un groupement :
- alkyle (C₂-C₄) linéaire ou ramifié substitué par un atome d'halogène ou un groupement alkoxy (C₁-C₆) linéaire ou ramifié, phénoxy, 2-tétrahydrofuryle ou cycloalkyle,
- cycloalkyle(C₃-C₇),
- phényle ou benzyle, chacun étant substitué par un atome d'halogène ou un groupement amino convenablement protégé,
R'₄ et R'₅ représente chacun un groupement alkyle (C₁-C₆) linéaire ou ramifié,
ou 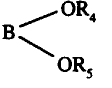 forme un ester boronique de pinanediol,
pour conduire :
- soit au composé de formle (I/a), cas particulier des composés de formule (I) : 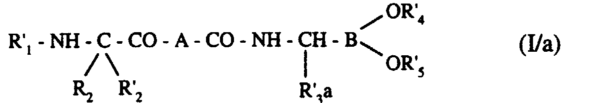 dans laquelle R'₁, R₂, R'₂, A, R'₄ et R'₅ ont la même signification que précédemment et R'_{3ₐ} représente un groupement alkyle (C₂-C₄) linéaire ou ramifié (substitué par un groupement alkoxy (C₁-C₆) linéaire ou ramifié, phénoxy, 2-tétrahydrofuryle ou cycloalkyle (C₃-C₇)) ou un groupement cycloalkyle (C₃-C₇),
- soit au composé de formule (VIII) : 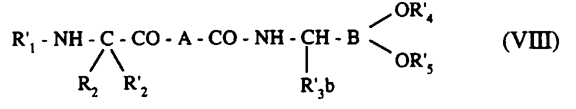 dans laquelle R'₁, R₂, R'₂, A, R'₄ et R'₅ ont la même signification que précédemment et R'_{3b} représente un groupement :
- alkyle (C₂-C₄) substitué par un atome d'halogène,
- phényle ou benzyle, chacun étant substitué par un atome d'halogène ou un groupement amino convenablement protégé,
composé de formule (VIII) :
° qui lorsque R'_{3b} représente un groupement :
- alkyle substitué par un atome d'halogène,
- phényle ou benzyle, chacun étant substitué par un atome d'halogène,
peut être transformé en dérivé cyano correspondant par action du cyanure de cuivre puis en dérivé amidino correspondant de formule (I/b), cas particulier des composés de formule (I), par réaction de l'ammoniaque : 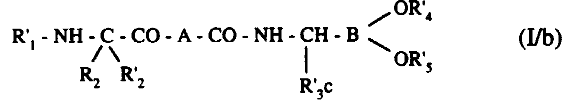 dans laquelle R'₁, R₂, R'₂, A, R'₄ et R'₅ ont la même signification que précédemment et R'_{3_{c}} représente un groupement alkyle, phényle ou benzyle, chacun étant substitué par un groupement amidino,
° qui lorsque R'_{3b} représente :
- un groupement alkyle substitué par un atome d'halogène peut être transformé en dérivé azido correspondant par action de l'azoture de sodium, puis par hydrogénation catalytique en dérivé amino correspondant de formule (I/c), cas particulier des composés de formule (I),
- ou, un groupement phényle ou benzyle, chacun étant substitué par un groupement amino protégé,
peut subir une déprotection pour conduire au composé de formule (I/c), cas particulier des composés de formule (I) : 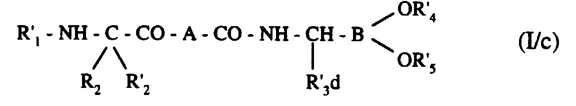
dans laquelle R'₁, R₂, R'₂, A, R'₄ et R'₅ ont la même signification que précédemment et R'_{3d} représente un groupement alkyle, phényle ou benzyle, chacun étant substitué par un groupement amino,
composé de formule (I/c),
dont on peut transformer le radical amino du groupement R'_{3d} en radical guanidino par réaction avec du cyanamide, pour conduire au composé de formule (I/d), cas particulier des composés de formule (I) : 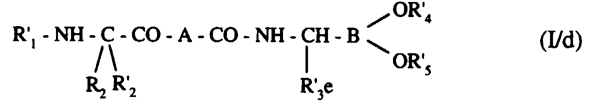 dans laquelle R'₁, R₂, R'₂, A, R'₄ et R'₅ ont la même signification que précédemment et R'₃ₑ représente un groupement alkyle, phényle ou benzyle, chacun étant substitué par un groupement guanidino,
composé de formule (I/a), (I/b), (I/c) ou (I/d) dont on déprotège, si on le souhaite, la fonction amine terminale et que l'on transforme, en milieu inerte, à l'aide de trichlorure de bore, en acide boronique de formule (I/e), cas particulier des composés de formule (I) : 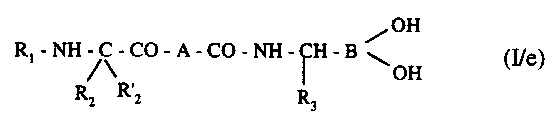 dans laquelle R₁, R₂, R'₂, A et R₃ ont la même signification que dans la formule (I),
composé de formule (I/a), (I/b), (I/c), (I/d) ou (I/e) :
- que l'on purifie éventuellement selon une technique classique de purification,
- dont on sépare, si on le souhaite, les énantiomères selon une technique classique de séparation
et que l'on transforme, le cas échéant, en ses sels d'addition à un acide ou une base pharmaceutiquement acceptable.

12. Compositions pharmaceutiques contenant comme principe actif au moins un composé selon l'une quelconques revendications 1 à 10, seul ou en combinaison avec un ou plusieurs véhicules inertes, non toxiques pharmaceutiquement acceptables.

13. Compositions pharmaceutiques selon la revendication 12 utiles en tant qu'inhibiteurs de trypsine-like serine proteases.

14. Compositions pharmaceutiques selon la revendication 12 utiles en tant qu'inhibiteurs de thrombine.

## Claims

1. Compounds of formula (I): 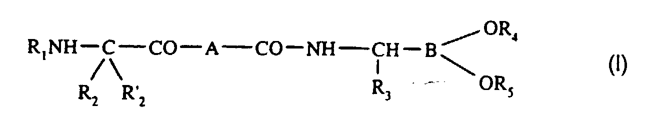 in which:
R₁ represents a hydrogen atom or a linear or branched (C₁-C₆)acyl, linear or branched (C₁-C₆)alkyl, benzyl, linear or branched (C₁-C₆)alkoxycarbonyl, benzyloxycarbonyl, phenoxycarbonyl, 5-[(dimethyl)amino]naphthylsulphonyl, alkoxycarbonylmethyl or carboxymethyl group,
R₂ represents a hydrogen atom or a group:
- phenyl,
- benzyl (unsubstituted or substituted on the phenyl ring by one or more halogen atoms or groups linear or branched (C₁-C₆)alkyl, linear or branched (C₁-C₆)alkoxy, hydroxy or bicyclooct-1-yl (unsubstituted or substituted by a hydroxy group or by a linear or branched (C₁-C₆)alkoxy group)),
- thienylmethyl,
- (pyridinyl)methyl,
- diphenylmethyl,
- fluorenyl,
- naphthylmethyl,
- benzocyclobutyl,
- (dicyclopropylmethyl)methyl,
- indanyl, or
- (C₃-C₇)cycloalkyl-methyl,
R'₂ represents a hydrogen atom or a benzyl group, or
R₂ and R'₂ together represent C₆H₅-CH=,
R₃ represents a group:
- linear or branched (C₂-C₄)alkyl substituted by a linear or branched (C₁-C₆)alkoxy, phenoxy, guanidino, amidino, amino, 2-tetrahydrofuryl or (C₃-C₇)cycloalkyl group,
- guanidinophenyl, amidinophenyl, aminophenyl,
- guanidinobenzyl, amidinobenzyl, aminobenzyl, or
- (C₃-C₇)cycloalkyl,
R₄ and R₅ each represents a hydrogen atom or a linear or branched (C₁-C₆)alkyl group,
or 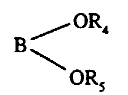 forms a boronic ester of pinanediol,
A represents any one of the following groups:
* 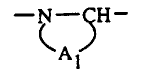
wherein:
A₁, with the nitrogen and carbon atoms to which it is bonded, represents a cyclic structure selected from the following structures:
- perhydroindole,
- 2-azabicyclo[2.2.2]octane,
- 2-azabicyclo[3.3.0]octane,
- 2-azabicyclo[2.2.1]heptane,
- perhydroisoindole,
- indoline,
- isoindoline,
- perhydroquinoline,
- perhydroisoquinoline,
- 1,2,3,4-tetrahydroquinoline,
- 1,2,3,4-tetrahydroisoquinoline,
- thiazolidine optionally substituted by one or more linear or branched (C₁-C₆)alkyl groups,
* 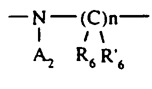
wherein:
n represents 1 or 2,
R₆ represents a hydrogen atom or a (C₃-C₇)cycloalkyl, linear or branched (C₁-C₆)alkyl or carboxymethyl group,
R'₆ represents a hydrogen atom,
or
R₆ and R'₆, with the carbon atom carrying them, form a (C₃-C₇)cycloalkyl group, A₂ represents a group linear or branched (C₁-C₆)alkyl, phenyl, indanyl, (C₃-C₇)cycloalkyl (unsubstituted or substituted by one or more methyl groups), (C₃-C₇)cycloalkenyl, cyclopentadienyl, 2,2,2-trifluoro-1-cyclopentylethyl, bicyclo[2.1.1]hexyl, bicyclo[2.2.1]heptyl or a group: 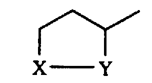 wherein
X and Y are different and represent an oxygen atom, a sulphur atom, an NH group or a CH₂- group,
or
A₂ represents a hydrogen atom (with the proviso that, in that case, R₆ represents a (C₃-C₇)cycloalkyl group and R'₆ represents a hydrogen atom, or R₆ and R'₆, with the carbon atom carrying them, form a (C₃-C₇)cycloalkyl group),
their enantiomers, diastereoisomers and epimers, and addition salts thereof with a pharmaceutically acceptable acid or base.

2. Compounds of formula (I) according to claim 1 in which R₁ represents an acetyl group, their enantiomers, diastereoisomers and epimers, and addition salts thereof with a pharmaceutically acceptable acid or base.

3. Compounds of formula (I) according to claim 1 in which R₂ represents a benzyl group, their enantiomers, diastereoisomers and epimers, and addition salts thereof with a pharmaceutically acceptable acid or base.

4. Compounds of formula (I) according to claim 1 in which A represents a 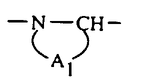 group as defined in claim 1, their enantiomers, diastereoisomers and epimers, and addition salts thereof with a pharmaceutically acceptable acid or base.

5. Compounds of formula (I) according to claim 1 in which A represents a 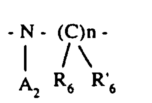 group as defined in claim 1, their enantiomers, diastereoisomers and epimers, and addition salts thereof with a pharmaceutically acceptable acid or base.

6. Compounds of formula (I) according to claim 4 in which A₁, with the nitrogen and carbon atoms to which it is bonded, represents a perhydroindole ring, their enantiomers, diastereoisomers and epimers, and addition salts thereof with a pharmaceutically acceptable acid or base.

7. Compounds of formula (I) according to claim 1 in which R₃ represents a linear or branched (C₂-C₄)guanidinoalkyl group, their enantiomers, diastereoisomers and epimers, and addition salts thereof with a pharmaceutically acceptable acid or base.

8. Compounds of formula (I) according to claim 1 in which R₃ represents a (C₃-C₇)-cycloalkyl group, their enantiomers, diastereoisomers and epimers, and addition salts thereof with a pharmaceutically acceptable acid or base.

9. Compound of formula (I) according to claim 1 which is 1-(R)-[(Ac-(R)Phe-Phi)-amino]-4-guanidinobutylboronic acid, wherein Ac represents acetyl, (R)-Phe represents (R)-phenylalanyl and Phi represents (2S,3aS,7aS)-perhydroindole-2-carbonyl, its isomers, and addition salts thereof with a pharmaceutically acceptable acid or base.

10. Compound of formula (I) according to claim 1 which is 1-(R)-{[Ac-Phe-(N-cyclopentyl)Gly]amino}-4-guanidinobutylboronic acid acetate, wherein Ac represents acetyl, Phe represents phenylalanyl having the (R,S) or (R) configuration and Gly represents glycyl, its enantiomers, and addition salts thereof with a pharmaceutically acceptable acid or base.

11. Process for the preparation of compounds of formula (I) according to claim 1, characterised in that a protected amino acid of formula (II), which has optionally been separated into its isomers by a conventional separating technique: 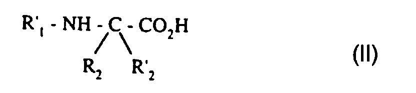 in which:
R'₁ represents a linear or branched (C₁-C₆)acyl, benzyl, linear or branched (C₁-C₆)-alkoxycarbonyl, benzyloxycarbonyl or phenoxycarbonyl group, and
R₂ and R'₂ are as defined for formula (I),
is reacted in accordance with a peptide coupling technique with a second protected amino acid of formula (III), which has optionally been separated into its isomers by a conventional separating technique:
HA-CO₂CH₂C₆H₅ (III)
in which A is as defined for formula (I), to yield a compound of formula (IV): 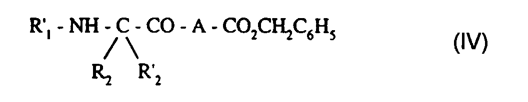 in which R'₁, R₂, R'₂ and A are as defined above,
the acid function of which is deprotected by catalytic hydrogenation to yield a compound of formula (V): 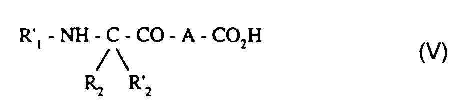 in which R'₁, R₂, R'₂ and A are as defined above,
which is reacted with N-hydroxysuccinimide in the presence of 1,3-dicyclohexylcarbodiimide, in an anhydrous medium, to yield a compound of formula (VI): 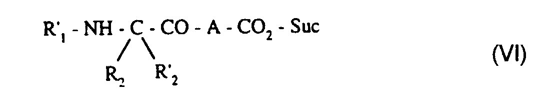 in which R'₁, R₂, R'₂ and A are as defined above and Suc represents a succinimido radical,
which is reacted in a basic medium with a compound of formula (VII): 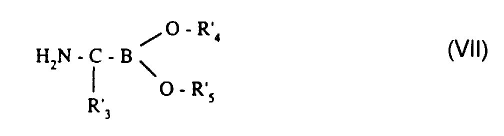 in which:
R'₃ represents a group:
- linear or branched (C₂-C₄)alkyl substituted by a halogen atom or by a linear or branched (C₁-C₆)alkoxy, phenoxy, 2-tetrahydrofuryl or cycloalkyl group,
- (C₃-C₇)cycloalkyl,
- phenyl or benzyl, each substituted by a halogen atom or by a suitably protected amino group,
R'₄ and R'₅ each represents a linear or branched (C₁-C₆)alkyl group, or 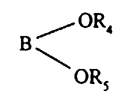
forms a boronic ester of pinanediol,
to yield:
- either a compound of formula (I/a), a particular case of the compounds of formula (I): 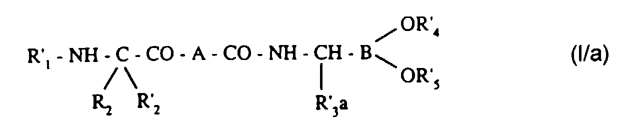 in which R'₁, R₂, R'₂, A, R'₄ and R'₅ are as defined above and R'₃ₐ represents a linear or branched (C₂-C₄)alkyl group (substituted by a linear or branched (C₁-C₆)alkoxy, phenoxy, 2-tetrahydrofuryl or (C₃-C₇)cycloalkyl group) or a (C₃-C₇)cycloalkyl group,
- or a compound of formula (VIII): 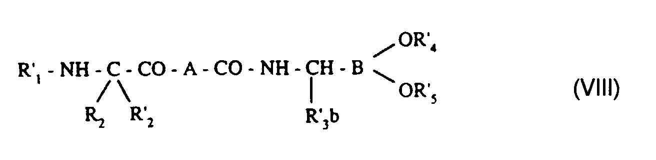 in which R'₁, R₂, R'₂, A, R'₄ and R'₅ are as defined above and R'_{3b} represents a group:
- (C₂-C₄)alkyl substituted by a halogen atom,
- phenyl or benzyl, each substituted by a halogen atom or by a suitably protected amino group,
which compound of formula (VIII):
° when R'_{3b} represents a group:
- alkyl substituted by a halogen atom,
- phenyl or benzyl, each substituted by a halogen atom,
may be converted into the corresponding cyano compound by the action of copper cyanide and then into the corresponding amidino compound of formula (I/b), a particular case of the compounds of formula (I), by the action of ammonium hydroxide: 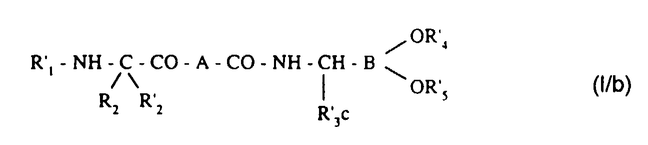 in which R'₁, R₂, R'₂, A, R'₄ and R'₅ are as defined above and R'_{3c} represents an alkyl, phenyl or benzyl group, each substituted by an amidino group,
° when R'_{3b} represents:
- an alkyl group substituted by a halogen atom, may be converted into the corresponding azide compound by the action of sodium azide and then, by catalytic hydrogenation, into the corresponding amino compound of formula (I/c), a particular case of the compounds of formula (I),
- or a phenyl or benzyl group, each substituted by a protected amino group, may undergo deprotection to yield a compound of formula (I/c), a particular case of the compounds of formula (I) 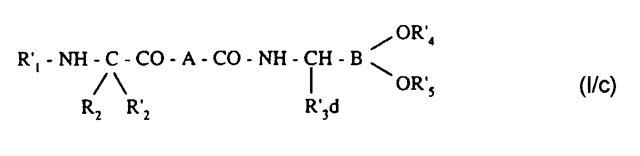
in which R'₁, R₂, R'₂, A, R'₄ and R'₅ are as defined above and R'_{3d} represents an alkyl, phenyl or benzyl group, each substituted by an amino group,
in which compound of formula (I/c)
the amino radical of the R'_{3d} group may be converted into a guanidino radical by reaction with cyanamide, to yield a compound of formula (I/d), a particular case of the compounds of formula (I): 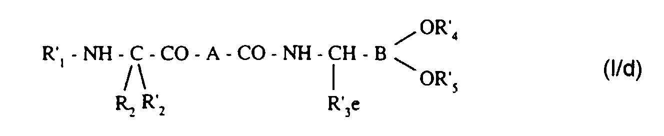 in which R'₁, R₂, R'₂, A, R'₄ and R'₅ are as defined above and R'₃ₑ represents an alkyl, phenyl or benzyl group, each substituted by a guanidino group,
the terminal amine function of which compound of formula (I/a), (I/b), (I/c) or (I/d) is deprotected, if desired, and which compound is converted, in an inert medium, with the aid of boron trichloride, into a boronic acid of formula (I/e), a particular case of the compounds of formula (I): 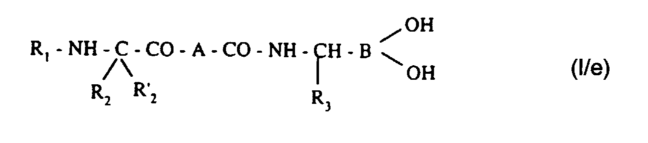 in which R₁, R₂, R'₂, A and R₃ are as defined for formula (I),
which compound of formula (I/a), (I/b), (I/c), (I/d) or (I/e):
- is purified, where appropriate, by a conventional purification technique,
- is, if desired, separated into its enantiomers by a conventional separating technique,
and is converted, where appropriate, into its addition salts with a pharmaceutically acceptable acid or base.

12. Pharmaceutical compositions comprising as active ingredient at least one compound according to any one of claims 1 to 10, on its own or in combination with one or more inert, non-toxic, pharmaceutically acceptable carriers.

13. Pharmaceutical compositions according to claim 12 for use as inhibitors of trypsin-like serine proteases.

14. Pharmaceutical compositions according to claim 12 for use as inhibitors of thrombin.

## Patentansprüche

1. Verbindungen der Formel (I): 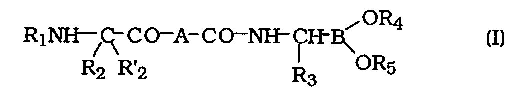 in der:
R₁ ein Wasserstoffatom oder eine geradkettige oder verzweigte (C₁-C₆)-Acylgruppe, geradkettige oder verzweigte (C₁-C₆)-Alkylgruppe, Benzylgruppe, geradkettige oder verzweigte (C₁-C₆)-Alkoxycarbonylgruppe, Benzyloxycarbonylgruppe, Phenyloxycarbonylgruppe, 5-[(Dimethyl)-amino-naphthylsulfonylgruppe, Alkoxycarbonylmethylgruppe oder Carboxymethylgruppe,
R₂ ein Wasserstoffatom oder eine der folgenden Gruppen:
- Phenyl,
- Benzyl (gegebenenfalls am Phenylkern substituiert durch eines oder mehrere Halogenatome oder geradkettige oder verzweigte (C₁-C₆)-Alkylgruppen, geradkettige oder verzweigte (C₁-C₆)-Alkoxygruppen, Hydroxygruppen oder Bicyclooct-1-yl-gruppen (die gegebenenfalls durch eine Hydroxygruppe oder eine geradkettige oder verzweigte (C₁-C₆)-Alkoxygruppe substituiert ist),
- Thienylmethyl,
- (Pyridinyl)-methyl,
- Diphenylmethyl,
- Fluorenyl,
- Naphthylmethyl,
- Benzocyclobutyl,
- (Dicyclopropylmethyl)-methyl,
- Indanyl oder
- Cycloalkyl-(C₃-C₇)-methyl,
R'₂ ein Wasserstoffatom oder eine Benzylgruppe oder
R₂ und R'₂ gemeinsam C₆H₅-CH=,
R₃ eine der folgenden Gruppen:
- geradkettiges oder verzweigtes (C₂-C₄)-Alkyl, welches durch eine geradkettige oder verzweigte (C₁-C₆)-Alkoxygruppe, Phenoxygruppe, Guanidinogruppe, Amidinogruppe, Aminogruppe, 2-Tetrahydrofurylgruppe oder (C₃-C₇)-Cycloalkylgruppe substituiert ist,
- Guanidinophenyl, Amidinophenyl, Aminophenyl,
- Guanidinobenzyl, Amidinobenzyl, Aminobenzyl oder
- (C₃-C₇)-Cycloalkyl,
R₄ und R₅ jeweils ein Wasserstoffatom oder eine geradkettige oder verzweigte (C₁-C₆)-Alkylgruppe
oder 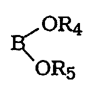 einen Pinandiol-boronsäureester bilden, und
A eine der folgenden Gruppen bedeuten:
* 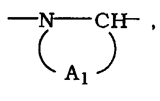
in der:
A₁ zusammen mit den Stickstoff- und Kohlenstoffatomen, an die sie gebunden ist, eine cyclische Struktur darstellt ausgewählt aus den folgenden Strukturen:
- Perhydroindol,
- 2-Azabicyclo[2.2.2]octan,
- 2-Azabicyclo[3.3.0]octan,
- 2-Azabicyclo[2.2.1]heptan,
- Perhydroisoindol,
- Indolin,
- Isoindolin,
- Perhydrochinolin,
- Perhydroisochinolin,
- 1,2,3,4-Tetrahydrochinolin,
- 1,2,3,4-Tetrahydroisochinolin,
- Thiazolidin, welches gegebenenfalls durch eine oder mehrere geradkettige oder verzweigte (C₁-C₆)-Alkylgruppen substituiert ist,
- 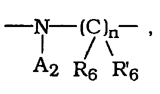
in der:
n 1 oder 2 darstellt,
R₆ ein Wasserstoffatom, eine (C₃-C₇)-Cycloalkylgruppe, eine geradkettige oder verzweigte (C₁-C₆)-Alkylgruppe oder eine Carboxymethylgruppe darstellt,
R'₆ ein Wasserstoffatom bedeutet
oder
R₆ und R'₆ gemeinsam mit dem sie tragenden Kohlenstoffatom eine (C₃-C₇)-Cycloalkylgruppe darstellen,
A₂ eine geradkettige oder verzweigte (C₁-C₆)-Alkylgruppe, Phenylgruppe, Indanylgruppe, (C₃-C₇)-Cycloalkylgruppe (die gegebenenfalls durch eine oder mehrere Methylgruppen substituiert ist), (C₃-C₇)-Cycloalkenylgruppe, Cyclopentadienylgruppe, 2,2,2-Trifluor-1-cyclopentylethylgruppe, Bicyclo[2.1.1]hexylgruppe, Bicyclo[2.2.1]heptylgruppe oder eine Gruppe der Formel: 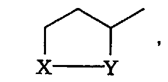 in der:
X und Y, die voneinander verschieden sind, ein Sauerstoffatom, Schwefelatom, eine NH-Gruppe oder eine CH₂-Gruppe darstellen, bedeutet,
oder
A₂ ein Wasserstoffatom (mit der Maßgabe, daß in diesem Fall R₆ eine (C₃-C₇)-Cycloalkylgruppe und R'₆ ein Wasserstoffatom bedeuten oder R₆ und R'₆ gemeinsam mit dem sie tragenden Kohlenstoffatom eine (C₃-C₇)-Cycloalkylgruppe darstellen) bedeutet,
deren Enantiomere, Diastereoisomere und Epimere sowie deren Additionssalze mit einer pharmazeutisch annehmbaren Säure oder Base.

2. Verbindungen der Formel (I) nach Anspruch 1, in der R₁ eine Acetylgrupe darstellt, deren Enantiomere, Diastereoisomere und Epimere sowie deren Additionssalze mit einer pharmazeutisch annehmbaren Säure oder Base.

3. Verbindungen der Formel (I) nach Anspruch 1, worin R₂ eine Benzylgruppe darstellt, deren Enantiomere, Diastereoisomere und Epimere sowie deren Additionssalze mit einer pharmazeutisch annehmbaren Säure oder Base.

4. Verbindungen der Formel (I) nach Anspruch 1, in der A eine Gruppe der Formel 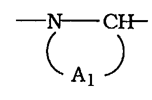 darstellt, wie sie in Anspruch 1 definiert ist, deren Enantiomere, Diastereoisomere und Epimere sowie deren Additionssalze mit einer pharmazeutisch annehmbaren Säure oder Base.

5. Verbindungen der Formel (I) nach Anspruch 1, in der A eine Gruppe der Formel 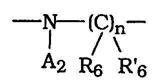 darstellt, wie sie in Anspruch 1 definiert ist, deren Enantiomere, Diastereoisomere und Epimere sowie deren Additionssalze mit einer pharmazeutisch annehmbaren Säure oder Base.

6. Verbindungen der Formel (I) nach Anspruch 4, in der A₁ mit den Stickstoff- und Kohlenstoffatomen, an die sie gebunden ist, einen Perhydroindolring bildet, deren Enantiomere, Diastereoisomere und Epimere sowie deren Additionssalze mit einer pharmazeutisch annehmbaren Säure oder Base.

7. Verbindungen der Formel (I) nach Anspruch 1, in der R₃ eine geradkettige oder verzweigte Guanidino-(C₂-C₄)-alkylgruppe darstellt, deren Enantiomere, Diastereoisomere und Epimere sowie deren Additionssalze mit einer pharmazeutisch annehmbaren Säure oder Base.

8. Verbindungen der Formel (I) nach Anspruch 1, in der R₃ eine (C₃-C₇)-Cycloalkylgruppe darstellt, deren Enantiomere, Diastereoisomere und Epimere sowie deren Additionssalze mit einer pharmazeutisch annehmbaren Säure oder Base.

9. Verbindung der Formel (I) nach Anspruch 1, nämlich 1-(R)-[(Ac-(R)Phe-Phi)-amino]-4-guanidinobutyl-boronsäure, worin Ac für Acetyl, (R)Phe für (R)-Phenylalanyl, Phi für (2S,3aS,7aS)-Perhydroindol-2-carbonyl stehen, deren Isomere und deren Additionssalze mit einer pharmazeutisch annehmbaren Säure oder Base.

10. Verbindung der Formel (I) nach Anspruch 1, nämlich 1-(R)-{[Ac-Phe-(N-cyclopentyl)-Gly]-amino}-4-guanidinobutyl-boronsäure, Acetat, worin Ac für Acetyl, Phe für Phenylalanyl der Konfiguration (R,S) oder (R) und Gly für Glycyl stehen, deren Isomere sowie deren Additionssalze mit einer pharmazeutisch annehmbaren Säure oder Base.

11. Verfahren zur Herstellung der Verbindungen der Formel (I) nach Anspruch 1, dadurch gekennzeichnet, daß man eine geschützte Aminosäure der Formel (II), die man gegebenenfalls mit Hilfe einer klassischen Trennmethode in die Isomeren aufgetrennt hat: 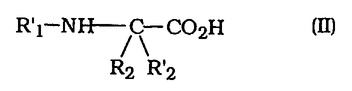 in der:
R'₁ eine geradkettige oder verzweigte (C₁-C₆)-Acylgrupe, Benzylgruppe, geradkettige oder verzweigte (C₁-C₆)-Alkoxycarbonylgruppe, Benzyloxycarbonylgruppe oder Phenoxycarbonylgruppe bedeutet
und
R₂ und R'₂ die bezüglich der Formel (I) angegebenen Bedeutungen besitzen, mit Hilfe einer Peptidkupplungstechnik mit einer zweiten geschützten Aminosäure der Formel (III), die man gegebenenfalls mit Hilfe einer klassischen Trennmethode in ihre Isomeren aufgetrennt hat:
HA - CO₂CH₂C₆H₅ (III)
in der A die bezüglich der Formel (I) angegebenen Bedeutungen besitzt, zur Bildung der Verbindung der Formel (IV) umsetzt: 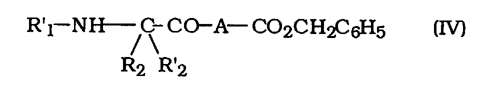 in der R'₁, R₂, R'₂ und A die oben angegebenen Bedeutungen besitzen,
von der man die Schutzgruppe der Säurefunktion durch katalytische Hydrierung abspaltet zur Bildung der Verbindung der Formel (V): 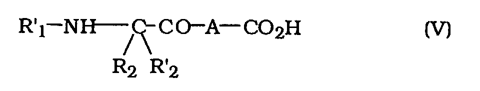 in der R'₁, R₂, R'₂ und A die oben angegebenen Bedeutungen besitzen,
welche man mit N-Hydroxysuccinimid in Gegenwart von 1,3-Dicyclohexylcarbodiimid in wasserfreiem Medium umsetzt zur Bildung der Verbindung der Formel (VI): 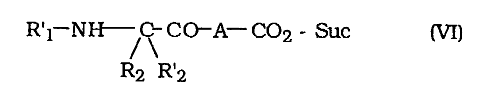 in der R'₁, R₂, R'₂ und A die oben angegebenen Bedeutungen besitzen und Suc für einen Succinimidorest steht,
welche man in basischem Medium mit einer Verbindung der Formel (VII): 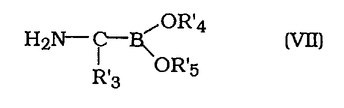 in der
R'₃ eine der folgenden Gruppen bedeutet:
- geradkettiges oder verzweigtes (C₂-C₄)-Alkyl, welches durch ein Halogenatom oder eine geradkettige oder verzweigte (C₁-C₆)-Alkoxygruppe, Phenoxygruppe, 2-Tetrahydrofurylgruppe oder Cycloalkylgruppe substituiert ist,
- (C₃-C₇)-Cycloalkyl,
- Phenyl oder Benzyl, welche jeweils durch ein Halogenatom oder eine in geeigneter Weise geschützte Aminogruppe substituiert sind,
R'₄ und R'₅ jeweils eine geradkettige oder verzweigte (C₁-C₆)-Alkylgruppe darstellen,
oder 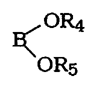 einen Pinandiol-boronsäureester bildet,
umsetzt zur Bildung:
- entweder der Verbindung der Formel (I/a), einem Sonderfall der Verbindungen der Formel (I): 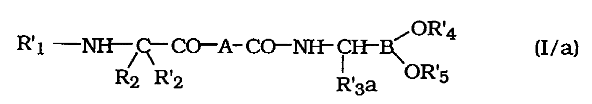 in der R'₁, R₂, R'₂, A, R'₄ und R'₅ die oben angegebenen Bedeutungen besitzen und R'₃ₐ eine geradkettige oder verzweigte (C₂-C₄)-Alkylgruppe (die durch eine geradkettige oder verzweigte (C₁-C₆)-Alkoxygruppe, Phenoxygruppe, 2-Tetrahydrofurylgruppe oder (C₃-C₇)-Cycloalkylgruppe substituiert ist) oder eine (C₃-C₇)-Cycloalkylgruppe darstellt,
- oder der Verbindung der Formel (VIII): 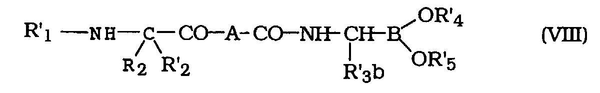 in der R'₁, R₂, R'₂, A, R'₄ und R'₅ die oben angegebenen Bedeutungen besitzen und R'_{3b} eine der folgenden Gruppen darstellt:
- (C₂-C₄)-Alkyl substituiert durch ein Halogenatom,
- Phenyl oder Benzyl, die jeweils durch ein Halogenatom oder eine in geeigneter Weise geschützte Aminogruppe substituiert sind,
welche Verbindung der Formel (VIII):
° wenn R'_{3b} eine der folgenden Gruppen darstellt:
- Alkyl substituiert durch ein Halogenatom.
- Phenyl oder Benzyl jeweils substituiert durch ein Halogenatom, durch Einwirkung von Kupfercyanid in das entsprechende Cyanoderivat und dann durch Einwirkung von Ammoniak in das entsprechende Amidinoderivat der Formel (I/b), einem Sonderfall der Verbindungen der Formel (I) umgewandelt werden kann: 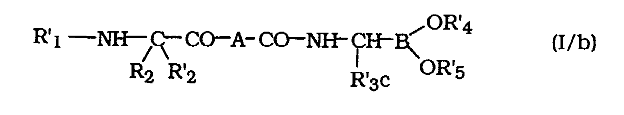 in der R'₁, R₂, R'₂, A, R'₄ und R'₅ die oben angegebenen Bedeutungen besitzen und R'_{3c} eine Alkyl-, Phenyl oder Benzylgruppe, die jeweils durch eine Amidinogruppe substituiert ist, darstellt,
° wenn R'_{3b} eine der folgenden Gruppen darstellt:
- Alkyl, substituiert durch ein Halogenatom, durch Einwirkung von Natriumazid in das entsprechende Azidoderivat und dann durch katalytische Hydrierung in das entsprechende Aminoderivat der Formel (I/c), einem Sonderfall der Verbindungen der Formel (I) umgewandelt werden kann,
- oder Phenyl- oder Benzyl, jeweils substituiert durch eine geschützte Aminogruppe, einer Behandlung zur Abspaltung der Schutzgruppe unterworfen werden kann zur Bildung der Verbindung der Formel (I/c), einem Sonderfall der Verbindungen der Formel (I): 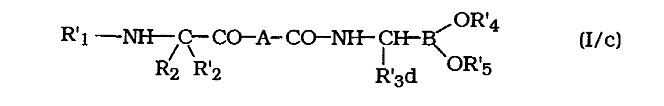 in der R'₁, R_{2, R}'2, A, R'₄ und R'₅ die oben angegebenen Bedeutungen besitzen und R'_{3d} eine Alkyl-, Phenyl oder Benzylgruppe, die jeweils durch eine Aminogruppe substituiert ist, darstellt,
von welcher
Verbindung der Formel (I/c)
man den Aminorest der Gruppe R'_{3d} durch Reaktion mit Cyanamid in einen Guanidinorest umwandeln kann zur Bildung der Verbindung der Formel (I/d), einem Sonderfall der Verbindungen der Formel (I): 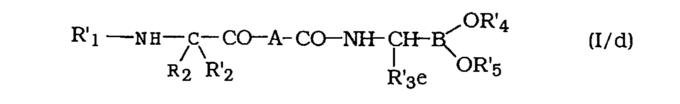 in der R'₁, R₂, R'₂, A, R'₄ und R'₅ die oben angegebenen Bedeutungen besitzen und R'₃ₑ eine Alkyl-, Phenyl- oder Benzylgruppe, die jeweils durch eine Guanidinogruppe substituiert ist, darstellt,
von welchen Verbindungen der Formeln (I/a), (I/b), (I/c) oder (I/d) man gewünschtenfalls die Schutzgruppe der endständigen Aminofunktion abspalten und in inertem Medium mit Hilfe von Bortrichlorid in die Boronsäure der Formel (I/e), einem Sonderfall der Verbindungen der Formel (I) umwandeln kann: 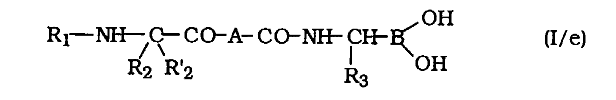 in der R₁, R₂, R'₂, A und R₃ die bezüglich der der Formel (I) angegebenen Bedeutungen besitzen,
welche Verbindungen der Formeln (I/a), (I/b), (I/c), (I/d) oder (I/e):
- man gegebenenfalls mit Hilfe einer klassischen Reinigungsmethode reinigt,
- gewünschtenfalls mit Hilfe einer klassischen Trennmethode in die Enantiomeren auftrennt oder
- gegebenenfalls in die Additionssalze mit einer pharmazeutisch annehmbaren Säure oder Base umwandelt.

12. Pharmazeutische Zubereitungen enthaltend als Wirkstoff mindestens eine Verbindung nach einem der Ansprüche 1 bis 10 allein oder in Kombination mit einem oder mehreren inerten, nichttoxischen, pharmazeutisch annehmbaren Trägermaterialien.

13. Pharmazeutische Zubereitungen nach Anspruch 12 geeignet als Inhibitoren von trypsinartigen Serinproteasen.

14. Pharmazeutische Zubereitungen nach Anspruch 12 geeignet als Thrombin-Inhibitoren.
